# EUROPEAN PATENT SPECIFICATION

(11) **EP 0 888 346 B1**
(45) Date of publication and mention of the grant of the patent: **06.06.2001**
(21) Application number: 97906466.4
(22) Date of filing: 11.02.1997
(51) Int. Cl.: C07D 455/02, A61K 31/47, C07D 491/14, C07D 217/16, C07D 491/04, C07D 405/04

(54) **CORALYNE ANALOGS AS TOPOISOMERASE INHIBITORS**
CORALYNANALOGE ALS TOPOISOMERASE INHIBITOREN
ANALOGUES DE CORALYNE UTILES EN TANT QU'INHIBITEURS DE TOPOISOMERASE

(30) Priority: 12.02.1996 US 11452 P; 01.10.1996 US 32161 P
(43) Date of publication of application: 07.01.1999
(73) Proprietor: RUTGERS, THE STATE UNIVERSITY OF NEW JERSEY, New Brunswick, NJ 08903 (US)
(72) Inventor: LAVOIE, Edmond, J., Princeton Junction, NJ 08550 (US); MAKHEY, Darshan, B., Lanexa, KS 66210 (US); LIU, Leroy, Fong, Bridgewater, NJ 08807 (US)
(74) Representative: Calamita, Roberto
(86) International application number: US9701676
(87) International publication number: WO9729106

(56) References cited:
- EP-A- 0 496 634
- US-A- 5 318 976
- CHEMICAL ABSTRACTS, vol. 124, no. 11, 11 March 1996 (1996-03-11) Columbus, Ohio, US; abstract no. 145854, SOTOMAYOR N ET AL: "Oxidation reactions of 2'-functionalized 3-aryltetrahydro- and 3,4-dihydroisoquinolines" XP002031075 & TETRAHEDRON, vol. 51, no. 46, 1995, pages 12721-12730,
- CHEMICAL ABSTRACTS, vol. 122, no. 7, 13 February 1995 (1995-02-13) Columbus, Ohio, US; abstract no. 081704, FITZGERALD J J ET AL: "Reaction of benzocyclobutene oxides with nitriles: synthesis of hypecumine and other 3-substituted isoquinolines " XP002031076 & TETRAHEDRON LETT., vol. 35, no. 49, 1994, pages 9191-9194,
- CHEMICAL ABSTRACTS, vol. 118, no. 7, 15 February 1993 (1993-02-15) Columbus, Ohio, US; abstract no. 059563, YAMAMOTO Y ET AL: "Reaction of 6H-1,3-oxazin-6-one with benzyne giving isoquinoline derivatives" XP002031077 & ANNU. REP. TOHOKU COLL. PHARM., vol. 38, 1991, pages 43-45,
- CHEMICAL ABSTRACTS, vol. 117, no. 13, 28 September 1992 (1992-09-28) Columbus, Ohio, US; abstract no. 131034, BADIA D ET AL: "Silicon-mediated isoquinoline synthesis: preparation and stereochemical characterization of 4-hydroxy-3-phenylisoquinolines" XP002031078 & TETRAHEDRON, vol. 48, no. 21, 1992, pages 4419-4430,
- CHEMICAL ABSTRACTS, vol. 117, no. 3, 20 July 1992 (1992-07-20) Columbus, Ohio, US; abstract no. 019892, MEMETZIDIS G ET AL: "Structure-affinity relationships of berbines or 5,6,13,13a-tetrahydro-8H-dibenzo[a,g]quino lizines at.alpha.-adrenoceptors" XP002031079 & EUR. J. MED. CHEM., vol. 26, no. 6, 1991, pages 605-611,
- CHEMICAL ABSTRACTS, vol. 115, no. 5, 5 August 1991 (1991-08-05) Columbus, Ohio, US; abstract no. 048721, NELSON J T ET AL: "Proton and carbon-13 NMR spectra of fifteen substituted isoquinolines" XP002031080 & MAGN. RESON. CHEM., vol. 29, no. 5, 1991, pages 513-517,
- CHEMICAL ABSTRACTS, vol. 112, no. 19, 7 May 1990 (1990-05-07) Columbus, Ohio, US; abstract no. 179554, SHCHERBAKOVA I V ET AL: "2-Benzopyrilium salts. 35. Synthesis of the natural alkaloid dehydronorcoralydine and other substituted salts of dibenzo[a,g]quinolizine" XP002031081 & KHIM. PRIR. SOEDIN., no. 1, 1989, pages 75-80,
- CHEMICAL ABSTRACTS, vol. 111, no. 13, 25 September 1989 (1989-09-25) Columbus, Ohio, US; abstract no. 115004, AGUIRRE J M ET AL: "Reaction of 1,2-diarylethylamides with ethyl polyphosphate (EPP): correlation of the von Braun, Ritter and Bischler-Napieralski reactions" XP002031082 & J. HETEROCYCL. CHEM., vol. 26, no. 1, 1989, pages 25-27,
- CHEMICAL ABSTRACTS, vol. 104, no. 19, 12 May 1986 (1986-05-12) Columbus, Ohio, US; abstract no. 168332, SCHIESS P ET AL: "Thermolytic ring opening of acyloxybenzocyclobutenes: an efficient route to 3-substituted isoquinolines" XP002031083 & TETRAHEDRON LETT., vol. 26, no. 33, 1985, pages 3959-3962,
- CHEMICAL ABSTRACTS, vol. 110, no. 25, 19 June 1989 (1989-06-19) Columbus, Ohio, US; abstract no. 231407, GARCIA A ET AL: "A simple direct approach to 1-substituted 3-arylisoquinolines deom deoxybenzoins and nitriles" XP002031084 & TETRAHEDRON, vol. 44, no. 21, 1988, pages 6681-6686,
- CHEMICAL ABSTRACTS, vol. 102, no. 1, 7 January 1985 (1985-01-07) Columbus, Ohio, US; abstract no. 006157, KITAMURA T ET AL: "Isoquinoline derivatives from the Ritter-type reaction of vinyl cations" XP002031085 & CHEM. LETT., no. 8, 1984, pages 1351-1354,
- CHEMICAL ABSTRACTS, vol. 104, no. 12, 24 March 1986 (1986-03-24) Columbus, Ohio, US; abstract no. 095573, WALTEROVA D ET AL: "Isolation, chemistry and biology of alkaloids from plants of Papaveraceae. Part XCV. Practical application of isotachophoresis in analysis of isoquinoline alkaloids" XP002031086 & ACTA UNIV. PALACKI. OLOMUC., FAC. MED., vol. 111, 1985, pages 23-36,
- CHEMICAL ABSTRACTS, vol. 101, no. 11, 10 September 1984 (1984-09-10) Columbus, Ohio, US; abstract no. 090742, DOMINGUEZ E ET AL: "Dehydrogention reactions of 1-substituted-3-aryltetrahydroisoquinoline derivatives" XP002031087 & J. HETEROCYCL. CHEM., vol. 21, no. 2, 1984, pages 525-528,
- CHEMICAL ABSTRACTS, vol. 97, no. 21, 22 November 1982 (1982-11-22) Columbus, Ohio, US; abstract no. 182180, QUAST U ET AL: "Heterocyclic.alpha.-carbinolamines with the isoquinuclidine skeleton. 3. Benzoisoquinuclidines" XP002031088 & LIEBIGS ANN. CHEM., no. 8, 1982, pages 1501-1508,
- CHEMICAL ABSTRACTS, vol. 96, no. 17, 26 April 1982 (1982-04-26) Columbus, Ohio, US; abstract no. 142656, SAFARYAN G P ET AL: "2-Benzopyrylium salts. 25. Reaction of 2-benzopyrylium salts with some nucleophiles" XP002031089 & KHIM. GETEROTSIKL. SOEDIN., no. 12, 1981, pages 1608-1611,
- CHEMICAL ABSTRACTS, vol. 89, no. 21, 20 November 1978 (1978-11-20) Columbus, Ohio, US; abstract no. 179810, BRADSHER C K ET AL: ".alpha.-Acyl-o-tolunitriles as intermediates in the preparation of 3-substituted isoquinolines and 1-amino-2-benzopyrylium derivatives" XP002031090 & J. ORG. CHEM., vol. 43, no. 20, 1978, pages 3817-3820,
- CHEMICAL ABSTRACTS, vol. 74, no. 15, 12 April 1971 (1971-04-12) Columbus, Ohio, US; abstract no. 76295, DOROFEENKO G N ET AL: "Synthesis of 3-aryl derivatives of 2-benzopyrylium salts with free.alpha.-positions" XP002031091 & KHIM. GETEROTSIKL. SOEDIN., no. 8, 1970, pages 1013-1014,
- CHEMICAL ABSTRACTS, vol. 125, no. 11, 9 September 1996 (1996-09-09) Columbus, Ohio, US; abstract no. 131653n, MAKHEY, DARSHAN ET AL: "Coralyne and related compounds as mammalian topoisomerase I and topoisomerase II poisons" XP002031092 & BIOORG. MED. CHEM., vol. 4, no. 6, 1996, pages 781-791,

## Description

### Background of the Invention

DNA topoisomerases represent a unique class of nuclear enzymes that alter the topological state of DNA by breaking and rejoining the phosphodiester backbone of DNA. Mammalian topoisomerase I is capable of altering the topology of DNA by transiently breaking one DNA strand, while topoisomerase II acts by causing double-strand breaks. Topoisomerase poisoning has recently been recognized as an attractive pharmacological target for the development of novel cancer chemotherapeutic agents. Alkaloids and their derivatives have been investigated as potential antitumor agents, including camptothecin and berberine (Hahn et al., *Antibiotics,* Vol. 3, Gottlieb et al. (eds.), Springer: New York, pp 577-584 (1975); Shideman, *Bull. Natl. Formulary Committee, 18*:3 (1950); Bhakkuni et al., *The Alkaloids;* Vol. 28, Brossi, A. (ed.), Academic Press: New York, pp 95-181 (1986); Suffness et al., *The Alkaloids;* Vol. XXV, Brossi, A. (ed.); Academic Press: New York, pp 178-197 (1985)).

Extensive studies of camptothecin and its derivatives have established that cellular topoisomerase I is the molecular target for the antitumor alkaloid camptothecin (Hsiang et al., *Cancer Res. 48*:1722-1726 (1988)). However, some lines of tumor cells have demonstrated resistance to camptothecin.

The demonstration that topoisomerase I is the molecular target for camptothecin has stimulated further identification and development of other topoisomerase I-targeting antitumor compounds (topoisomerase I poisons). Among these are actinomycin D, morpholinodoxorubicin, DNA minor groove binding bis- and tris-benzimidazoles, indolocarbazole derivatives, bulgarein, intoplicine, saintopin, indoloquinolinediones, nitidine derivatives and berberine derivatives. Many of these compounds are also dual poisons of both topoisomerase I and II. Although the number of new topoisomerase I poisons is increasing rapidly, except for camptothecin, topoisomerase I-poisoning has not been demonstrated to be responsible for cell killing for any of the new poisons. Similarly, it is unclear whether the intercalative mode of DNA binding, which is known to be essential for topoisomerase II poisoning, is responsible for poisoning topoisomerase I for the dual poisons.

Many studies have focused their attention on a widely distributed class of alkaloids, whose structures are related to the isoquinoline ring. Two of these compounds, the benzophenanthridine, nitidine, and the related alkaloid fagaronine have a high potency in inducing topoisomerase I-mediated DNA cleavage *in vitro* (Wang et al., *Chem. Res. Toxicol. 6*:813-818 (1993)). Several compounds belonging to the family of protoberberines, which are known for their antitumor activities in animals, have also been shown to be topoisomerase I poisons. Berberine represents one of the most intensively studied of the naturally-occurring protoberberine alkaloids and is reported to exhibit weak antitumor activity against P-388 leukemia in mice (Suffness et al., *The Alkaloids,* Vol. XXV, Brossi, A. (ed.), Academic Press: New York, pp 178-197 (1985)).

Coralyne, an alkaloid analog of protoberberine, has more pronounced antitumor activity relative to berberine, exhibiting significant activity *in vivo* in mice against L1210 and P388 leukemias (Zee-Cheng et al., *J. Med. Chem. 17*:347 (1974); Zee-Cheng et al., *J. Med. Chem. 19*:882 (1976)). While the molecular basis for its antitumor activity has not been identified, it has been speculated that the ability to bind to duplex and triplex DNA may contribute to the observed antileukemic activity (Wilson et al., *J. Med. Chem. 19*:1261 (1976) ; Lee et al., *Biochemistry 32*:5591-5597 (1993)). The antitumor activity of coralyne, coupled with its relatively low toxicity, prompted studies on the synthesis of a number of derivatives (Zee-Cheng et al., *J. Med. Chem. 17*:347 (1974)), and have suggested that the presence of the methyl substituent at the 8-position and unsaturation at the 5,6-position of coralyne are strongly associated with their antitumor activity against L1210 and P388 leukemias in mice (Messmer et al., *J*. *Pharm. Sci. 61*:1858-1859 (1972); Cushman et al., *J. Med. Chem. 28*:1031-1036 (1985); Stermitz et al., *J*. *Med. Chem. 18*:708-713 (1975); Janin et al., *J. Med. Chem. 36*:3686-3692 (1993)).

However, despite efforts to develop safe and effective therapeutics, the need exists for anti-cancer agents with improved cytotoxicity and which are effective against drug-resistant cancer cells.

### Summary of the Invention

The present invention provides compounds of the formula: 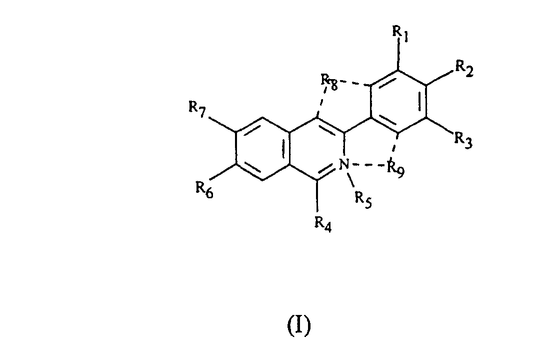 wherein R₁, R₂, R₆ and R₇ are independently selected from H, OH, and (C₁-C₈)alkoxy preferably -OCH₃; R₃ is OH or (C₁-C₈)alkoxy; R₄ is H
or (C₁-C₈)alkyl, preferably -OCH₃; R₅ is H,
(C₁-C₈) alkyl, preferably -CH₃, or is absent; and R₈ and R₉ are -CH=CH-, -(CH₂)₂ or absent. In some embodiments, R₃ may also be H.

Alternatively, R₂ and R₃ together are -OCH₂O-; R₁ and R₂ together are -OCH₂O-; R₆ and R₇ together are -OCH₂O-.

In one embodiment, R₂ and R₃ together are -OCH₂O-. According to a further embodiment, R₈ is -CH=CH-, R₉ is absent and R₃ is H. In another embodiment, R₉ is -CH=CH- or -(CH₂)₂, R₁ or R₂ is H, and R₅ and R₈ are absent. In yet a further embodiment, R₈ and R₉ are both absent.

The compounds of formula (I) show improved inhibitory activity against topoisomerase I, relative to the parent compounds, and are also effective inhibitors of topoisomerase II. The present compounds are also effective cytotoxic agents against cancer cells, and have cytotoxic activity against camptothecin-resistant human tumor cell lines.

Further provided are methods of inhibiting cancer cell growth comprising administering to a mammal afflicted with cancer an amount of the compound of formula (I), effective to inhibit the growth of such cancer cells. The compound or its salt may be administered in combination with a pharmaceutically acceptable carrier.

### Detailed Description

DNA topoisomerases are nuclear enzymes responsible for modifying the topological state of DNA. Analogs of coralyne were synthesized and evaluated for their activity as topoisomerase I and topoisomerase II poisons. These analogs were also evaluated for cytotoxicity against the human lymphoblast cell line, RPMI 8402, and its camptothecin-resistant variant, CPTK-5; the epidermal carcinoma cell line, KB 3-1; and gliobastomas (a CNS tumor), SF-268.

The pharmacological activity of these analogs exhibited a strong dependence on the substitution pattern and the nature of substituents. Several 1-benzylisoquinolines and 3-phenylisoquinolines were also synthesized. These compounds, which incorporate only a portion of the ring structure of coralyne, were evaluated as topoisomerase poisons and for cytotoxicity. These structure-activity studies indicate that the structural rigidity associated with the coralyne ring system may be critical for pharmacological activity. The presence of a 3,4-methylenedioxy substituent on these coralyne analogs was generally associated with enhanced activity as a topoisomerase poison. 5,6-Dihydro-3,4-methylenedioxy-10,11-dimethoxydibenzo[a,g]quinolizinium chloride was the most potent topoisomerase I poison among the coralyne analogs evaluated, having similar activity to camptothecin. This analog also possessed exceptional potency as a topoisomerase II poison.

According to the invention, cancer cells are inhibited by administration to a mammal afflicted with cancer of an effective amount of the compounds of Formula (I). As used herein, an "effective amount" is that amount which results in an inhibition of growth of the target cancer cells. As described herein, a suitable dose will be in the range of about 0.5 to about 100 mg/kg of body weight per day.

The compositions described herein are believed to be effective in the treatment of solid mammalian tumors or hematologic malignancies. These solid tumors include cancers of the head and neck, lung, mesothelioma, mediastinum, esophagus, stomach, pancreas, hepatobiliary system, small intestine, colon, rectum, anus, kidney, ureter, bladder, prostate, urethra, penis, testis, gynecological organs, ovarian, breast, endocrine system, skin and central nervous system; sarcomas of the soft tissue and bone; and melanoma of cutaneous and intraocular origin. Hematological malignancies include childhood leukemia and lymphomas, Hodgkin's disease, lymphomas of lymphocytic and cutaneous origin, acute and chronic leukemia, plasma cell neoplasm and cancers associated with AIDS. The preferred mammalian species for treatment are humans and domesticated animals.

Methods similar to those reported in the literature were used in the preparation of the dibenzo[*a,g*]quinolizinium derivatives (Zee-Cheng et al., *J. Pharm*. *Sci. 61*:969-971 (1972)). The general synthetic approach employed is outlined in Scheme 1. Reaction of the appropriately substituted dimethoxyphenethylamine with 3,4-dimethoxyphenylacetyl chloride provided the phenylacetamides, **5a-c,** which were cyclized in the presence of phosphorous oxychloride to the 3,4-dihydro-1-benzylisoquinoline intermediates, **6a-c.** These dihydroisoquinoline intermediates could be directly converted to the 5,6-dihydrodibenzo[*a,g*]quinolizinium derivatives, **7a-c,** using acetic anhydride in the presence fuming H₂SO₄. Alternatively, **6a-c** could be converted to **8a-c,** under Vilsmeier-Haack conditions (Le Quang et al., *Acad. Sc.,* ser. C 1340 (1968)). The dihydroisoquinoline intermediates, **6a-c,** could also be oxidized to their 1-benzylisoquinoline derivatives, **9a-c,** using palladium-on-carbon. Treatment of **9a-c** with acetic anhydride in the presence of H₂SO₄ provided the 8-methyldibenzo[*a,g*]quinolizinium derivatives, **2a-c.** Under Vilsmeier-Haack conditions, **9a-c** were converted to the dibenzo[*a*,*g*]quinolizinium chloride derivatives, **10a-c.**

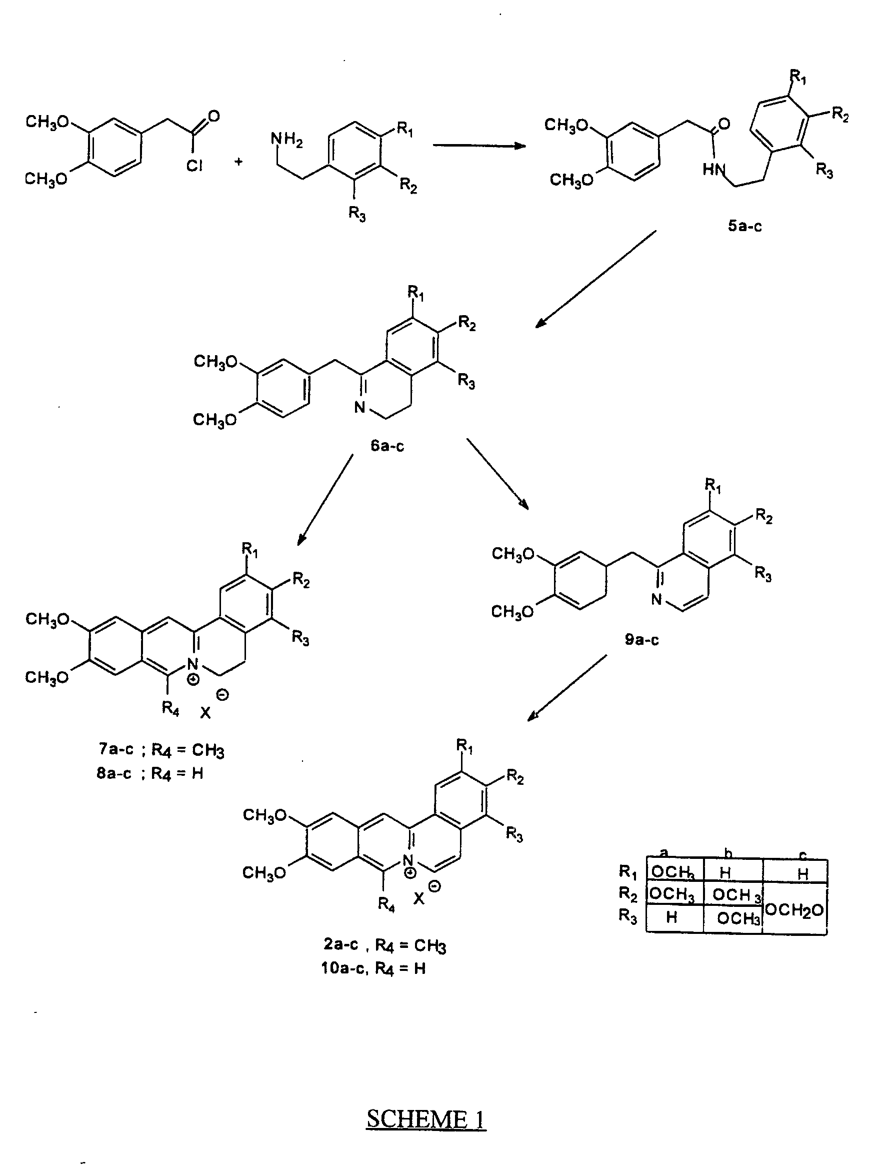

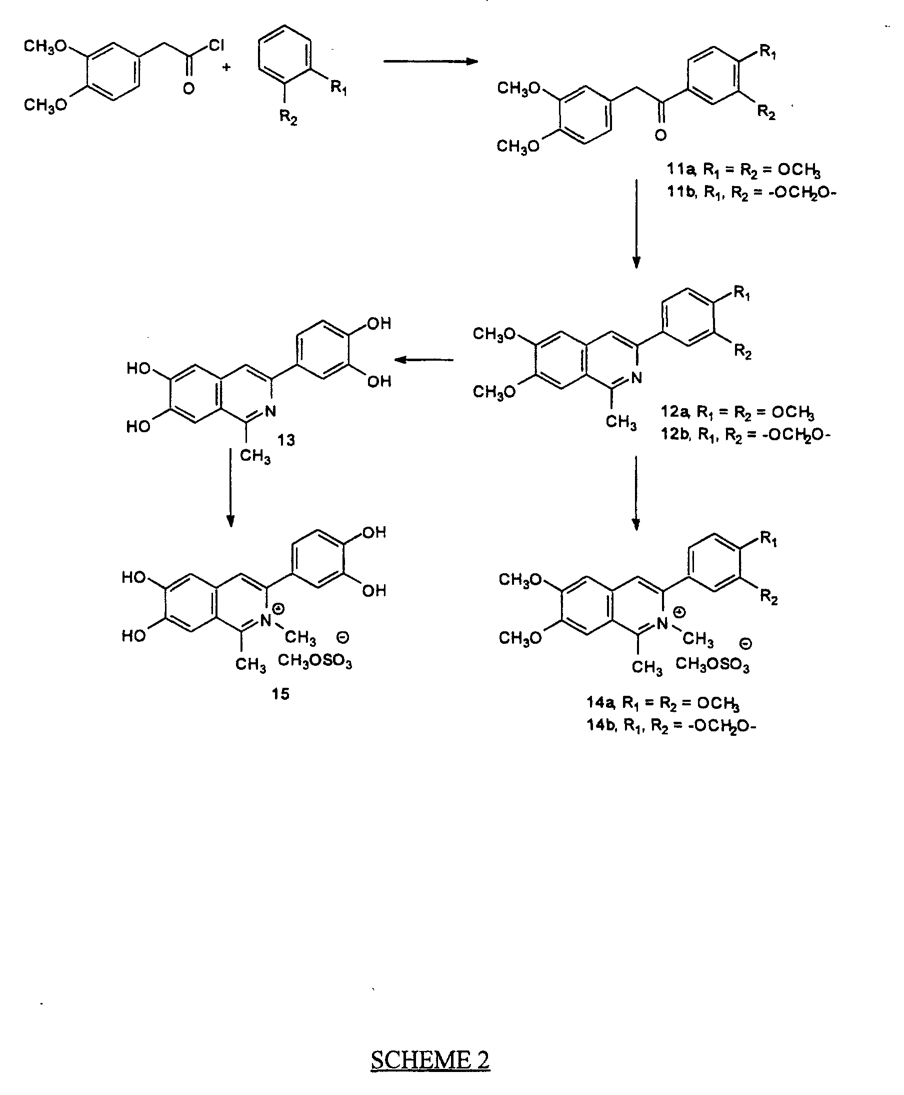

The synthetic methods employed for the preparation of the 1-methyl-3-phenylisoquinoline derivatives are outlined in Scheme 2. Friedel-Crafts acylation of 1,2-dimethoxybenzene and 1,2-(methylenedioxy)benzene with 3,4-dimethoxyphenyl-acetyl chloride using a slight modification of the literature procedure provided the ketone intermediates, **11a** and **11b,** respectively. These ketones were converted to 6,7-dimethoxy-1-methyl-3-(3,4-dimethoxyphenyl)isoquinoline, **12a,** and 6,7-methylenedioxy-1-methyl-3-(3,4-dimethoxyphenyl)-isoquinoline, **12b,** by reaction with acetonitrile in the presence of P₂O₅.

Cleavage of the methoxy groups of **12a** was accomplished using borontribromide in chloroform to provide the tetrahydroxy derivative, **13.** Compounds **12a, 12b,** and **13** were converted to their 2-methylisoquinolinium derivatives, **14a, 14b,** and **15,** respectively, by treatment with dimethyl sulfate.

The relative activity of coralyne and related compounds as inhibitors of mammalian topoisomerase is listed in Table 1, in the Examples. As shown in this table, coralyne and several of its analogs had pronounced activity, in some instances comparable to camptothecin, as mammalian topoisomerase I poisons. Previous studies on the influence of structure of a series of coralyne derivatives against L1210 and P388 leukemias in mice revealed that the presence of 8-alkyl substitution, planarity, and structural rigidity are critical factors for antitumor activity. In this study analogs were evaluated which had methoxy substituents at sites which were equivalent to either the 2,3-positions **(2a, 7a, 8a, 9a, 10a)** or 3,4-positions **(2b, 7b, 8b, 9b, 10b)** of coralyne. In addition, 3,4-methylenedioxy-substituted analogs **(2c, 7c, 8c, 9c, 10c)** were also synthesized and their pharmacological activities evaluated. The methylenedioxy group at the 3,4-position of these coralyne analogs appears to be a common factor associated with their potency as topoisomerase poisons. The methylenedioxy derivatives, **2c, 7c, 8c,** and **10c,** not only retained topoisomerase I poisoning activity comparable to that of coralyne, but also possessed notable activity as topoisomerase II poisons. As there is no evidence to suggest that the molecular mechanism of topoisomerase I poisoning is related to that responsible for topoisomerase II poisoning, the observation that, for these analogs, enhanced activity is observed for both of these topoisomerases was unanticipated. These results are consistent, however, in view of the fact that this modification in substitution pattern and substituents allows these molecules to more closely resemble the alkoxy substitution pattern on the potent topoisomerase I and II poison, nitidine.

In evaluating the relative potency of a series of coralyne analogs-as topoisomerase poisons, several differences were also noted in those structural requirements which favored activity when compared to the structure-activity relationships reported for the *in vivo* antitumor activity of varied coralyne derivatives. It is of particular interest that the best topoisomerase I poison in this series of compounds (**8c**) lacks an 8-alkyl substituent, which is not consistent with the structure-activity studies on antitumor activity in mice. The potent activity of **8c** is evident by the total cleavage of DNA observed in the presence of topoisomerase I which occurred at almost equivalent concentration for **8c** (0.27 *µ*M, 0.1 *µ*g/mL) and camptothecin (0.29 *µ*M, 0.1 *µ*g/mL).

In addition, **8c** possesses a kink in an otherwise planar molecule because of its 5,6-position being saturated. In previous studies on the antitumor activity of coralyne derivatives in mice, the presence of unsaturation at this position was associated with diminished activity. All other similarly substituted molecules **2c, 7c,** and **10c,** which are either planar and/or have an 8-methyl substituent, are 10-fold less active compared to **8c,** as topoisomerase I poisons. Berberine, which is a positional isomer of **8c,** is inactive when assayed under identical conditions as a topoisomerase I poison. Compounds **2b, 7b, 8b,** and **10b** did not exhibit significant activity as topoisomerase I poisons. Thus, relative to a 3,4-methylenedioxy substituent, dimethoxy substitution at 3,4-position of these coralyne analogs dramatically altered their potency as topoisomerase I poisons. While **8c** did exhibit modest activity as a topoisomerase II poison, several of these analogs, including **2c** and **10c,** did exhibit potent activity as topoisomerase II poisons (Table 1).

All of the 2,3-dimethoxy coralyne derivatives, with the exception of **10a,** are selective topoisomerase I poisons with no significant activity as topoisomerase II poisons. In contrast to the results observed with **2a, 7a,** and **8a,** compound **10a** was inactive as a topoisomerase I poison and did exhibit significant activity against topoisomerase II. The basis for this exceptional difference in activity associated with the ability of **10a** as a topoisomerase poison and the other 2,3-dimethoxy coralyne derivatives is unclear at this time.

Structural rigidity may be a critical requirement for retention of activity as topoisomerase poisons within this series of compounds. Compounds **9a, 9b,** and **9c** may be regarded as ring-opened analogs of coralyne which lack the carbon at the 8-position as well as the cationic charge associated with the quaternary ammonium group. All three of these analogs exhibited very weak activity as topoisomerase I or II poisons.

The 1-methyl-3-phenyl isoquinoline derivatives **12a** and **12b** and their N-methylated quaternized analogs **14a** and **14b** may be regarded as ring-opened analogs lacking C₅ -C₆ moiety of coralyne did not exhibit significant topoisomerase poisoning activity. Also, compounds **13** and **15** which are polyphenolic analogs of compounds **12a/12b** and **14a/14b**, respectively, were relatively ineffective as topoisomerase I or II poisons.

The cytotoxicity of coralyne and these analogs against the human lymphoblast cell line, RPMI 8402, is summarized in Table 1. No clear correlation was observed between cytotoxicity, as determined using MTA assay, and potency as either a topoisomerase I or II poison. In contrast to camptothecin, these derivatives exists as cationic quaternary ammonium compounds. On the basis of the intrinsic activity of several of these derivatives to inhibit topoisomerase I, it is possible that the quaternary ammonium functionality associated with the structure of the structurally-rigid analogs of coralyne could be a major factor in limiting cellular absorption and attenuating their cytotoxic activity. Fused planar cationic aromatic ring system of coralyne and berberine have been shown to intercalate with DNA. Furthermore, coralyne has been shown to bind to DNA triplexes. The extent to which these processes are related to poisoning of topoisomerases and the role of the cationic moiety in these molecular interactions has not been fully established.

Several of the analogs synthesized and evaluated for cytotoxicity in this study which were inactive as topoisomerase poisons did exhibit significant cytotoxicity. Specifically, compounds **9c, 13, 14b,** and **15** had appreciable cytotoxicity against RPMI 8402 cells. In these instances, the specific pharmacological target associated with their cytotoxicity is unknown.

The cytotoxic activity of these compounds was also evaluated in the camptothecin-resistant cell line, CPT-K5, which contains a mutant form of topoisomerase I. In viewing the cytotoxicity of the more potent topoisomerase I poisons in this study **(2a, 2c, 7a, 7c, 8a, 8c, 10c),** there were differences between the IC₅₀ values obtained for CPT-K5 cells as compared to those for RPMI 8402 cells. These data could be viewed as an indication that the pharmacological target associated with the cytotoxicity of these coralyne derivatives is related to their activity as topoisomerase I poisons. In view of similar differences, however, which can be noted for analogs which were inactive as topoisomerase I poison, one cannot ascribe this difference in effect specifically to the mutant form of topoisomerase I in CPT-K5. It also is evident that any difference in cytotoxicity between these cell lines for the compounds evaluated is minor relative to camptothecin.

This study demonstrates that minor structural variation among analogs of coralyne can have a profound impact on their activity as either topoisomerase I or topoisomerase II poisons. Coralyne analogs exhibited enhanced activity as both a topoisomerase I and II poison in those instances where there was a methylenedioxy substituent at the 3,4-position. These results are consistent with the potent activity of nitidine as a topoisomerase II and the spatial relationships of nitidine to these coralyne derivatives. In this study the analogs of coralyne which have potent activity as topoisomerase I poisons with little activity as topoisomerase II poisons had methoxy substituents at the 2,3-position. These data suggest that similarly substituted analogs of nitidine, i.e., 1,2-dimethoxy derivatives as opposed to its 2,3-methylenedioxy substituent, could exhibit similar selectivity with regard to its potential to inhibit either mammalian topoisomerase I or II.

Compound **7c** was also tested against the glioblastoma cell line SF-268. The results are presented in Table 2. The analog's effectiveness and selectivity against such tumor cells suggests that active transport may be occurring. Administration of analogs of the present invention by intrathecal injection could provide a means for achieving selective cytotoxicity against CNS tumors with little systemic toxicity.

Pharmaceutically acceptable salts of the biologically active compounds described herein may be used as well in practicing the claimed methods. Pharmaceutically acceptable salts may be formed using organic or inorganic bases, such as NaOH, Na(CO₃)₂, NaHCO₃, KOH and the like; as well as acids such as hydrochloric and sulfoacetic acids and the like.

Although the compounds described herein and/or their its salts may be administered as the pure chemicals, it is preferable to present the active ingredient as a pharmaceutical composition. The invention thus further provides the use of a pharmaceutical composition comprising one or more compounds and/or a pharmaceutically acceptable salt thereof, together with one or more pharmaceutically acceptable carriers therefor and, optionally, other therapeutic and/or prophylactic ingredients. The carrier(s) must be 'acceptable' in the sense of being compatible with the other ingredients of the composition and not deleterious to the recipient thereof.

Pharmaceutical compositions include those suitable for oral or parenteral (including intramuscular, subcutaneous and intravenous) administration. The compositions may, where appropriate, be conveniently presented in discrete unit dosage forms and may be prepared by any of the methods well known in the art of pharmacy. Such methods include the step of bringing into association the active compound with liquid carriers, solid matrices, semi-solid carriers, finely divided solid carriers or combination thereof, and then, if necessary, shaping the product into the desired delivery system.

Pharmaceutical compositions suitable for oral administration may be presented as discrete unit dosage forms such as hard or soft gelatin capsules, cachets or tablets each containing a predetermined amount of the active ingredient; as a powder or as granules; as a solution, a suspension or as an emulsion. The active ingredient may also be presented as a bolus, electuary or paste. Tablets and capsules for oral administration may contain conventional excipients such as binding agents, fillers, lubricants, disintegrants, or wetting agents. The tablets may be coated according to methods well known in the art., e.g., with enteric coatings.

Oral liquid preparations may be in the form of, for example, aqueous or oily suspension, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservative.

The compounds may also be formulated for parenteral administration (e.g., by injection, for example, bolus injection or continuous infusion) and may be presented in unit dose form in ampules, pre-filled syringes, small bolus infusion containers or in multi-does containers with an added preservative. The compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g., sterile, pyrogen-free water, before use.

For topical administration to the epidermis, the compounds may be formulated as ointments, creams or lotions, or as the active ingredient of a transdermal patch. Suitable transdermal delivery systems are disclosed, for example, in Fisher et al. (U.S. Patent No. 4,788,603) or Bawas et al. (U.S. Patent No. 4,931,279, 4,668,504 and 4,713,224). Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents. The active ingredient can also be delivered via iontophoresis, e.g., as disclosed in U.S. Patent Nos. 4,140,122, 4383,529, or 4,051,842.

Compositions suitable for topical administration in the mouth include unit dosage forms such as lozenges comprising active ingredient in a flavored base, usually sucrose and acacia or tragacanth; pastilles comprising the active ingredient in an inert base such as gelatin and glycerin or sucrose and acacia; mucoadherent gels, and mouthwashes comprising the active ingredient in a suitable liquid carrier.

When desired, the above-described compositions can be adapted to provide sustained release of the active ingredient employed, e.g., by combination thereof with certain hydrophilic polymer matrices, e.g., comprising natural gels, synthetic polymer gels or mixtures thereof.

The pharmaceutical compositions according to the invention may also contain other adjuvants such as flavorings, coloring, antimicrobial agents, or preservatives.

It will be further appreciated that the amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general, however, a suitable dose will be in the range of from about 0.5 to about 100 mg/kg, e.g., from about 10 to about 75 mg/kg of body weight per day, such as 3 to about 50 mg per kilogram body weight of the recipient per day, preferably in the range of 6 to 90 mg/kg/day, most preferably in the range of 15 to 60 mg/kg/day.

The compound is conveniently administered in unit dosage form; for example, containing 5 to 1000 mg, conveniently 10 to 750 mg, most conveniently, 50 to 500 mg of active ingredient per unit dosage form.

Ideally, the active ingredient should be administered to achieve peak plasma concentrations of the active compound of from about 0.5 to about 75 µM, preferably, about 1 to 50 µM, most preferably, about 2 to about 30 µM. This may be achieved, for example, by the intravenous injection of a 0.05 to 5% solution of the active ingredient, optionally in saline, or orally administered as a bolus containing about 1-100 mg of the active ingredient. Desirable blood levels may be maintained by continuous infusion to provide about 0.01-5.0 mg/kg/hr or by intermittent infusions containing about 0.4-15 mg/kg of the active ingredient(s).

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

The invention has been described with reference to various specific and preferred embodiments and techniques. However, it should be understood that many variations and modifications may be made while remaining within the spirit and scope of the invention.

The following examples are intended to illustrate but not limit the invention.

### EXAMPLES

### Example I - General.

Melting points were determined with a Thomas-Hoover Unimelt capillary melting point apparatus. Infrared spectral data (IR) were obtained on a Perkin-Elmer 1600 Fourier transform spectrophotometer and are reported in cm⁻¹. Proton (¹H NMR) and carbon (¹³C NMR) nuclear magnetic resonance were recorded on a Varian Gemini-200 Fourier Transform spectrometer at 200 MHz and 50 MHz, respectively. NMR spectra were recorded in CDCl₃ (unless otherwise noted) with chemical shifts reported in δ units downfield from tetramethylsilane (TMS). Coupling constants are reported in hertz. Mass spectra were obtained from Washington University Resource for Biomedical and Bio-Organic Mass Spectrometry. Column chromatography refers to flash chromatography conducted on SiliTech 32-63 µm, (ICN Biomedicals, Eschwegge, Ger.) using the solvent systems indicated. Combustion analyses were performed by Atlantic Microlabs, Inc., Norcross, GA.

### Example II - General procedure for the synthesis of 8-methyldibenzo-[a,g]quinolizinium acetosulfates (2a-c).

Fuming (20%) sulfuric acid (1 mL) was added to 4 mL of freshly distilled acetic anhydride resulting in a vigorous exothermic reaction and the mixture becoming wine-red in color. This mixture was heated at 85-90 °C for 10 min. A solution of 1-benzylisoquinoline **(9a-c,** 2.35 mmol in 1 mL of freshly distilled acetic anhydride), was then added under nitrogen to the wine red sulfuric acid solution and the resulting mixture was heated at 85-90 °C for 30-60 min. The reaction mixture was then cooled to room temperature and 5 mL methanol was added dropwise and allowed to stir for 30 min. The mixture was then chilled in an ice bath and stirred for an additional 30 min. The solid product obtained was filtered and washed successively twice with 2 mL distilled water, twice with 2 mL methanol and twice with 10 mL ether. The crude product was then recrystallized from hot methanol to provide 90-95% yield of the desired products as a yellow crystalline material.

*8-Methyl-2,3,10,11-Tetramethoxydibenzo[a,g]quinolizinium acetosulfate* ***(2a)*:** prepared from **9a**; mp 280 °C; IR 1735; ¹H NMR (DMSO-*d*₆) δ 3.28 (s, 3H), 3.94 (s, 3H), 4.03 (s, 3H), 4.09 (s, 6H), 7.42 (s, 1H), 7.53 (s, 1H), 7.80 (d, 1H, *J* = 8.0), 7.98 (s, 1H), 8.70 (d, 1H, *J* = 8.0), 9.28 (s, 1H); ¹³C NMR (DMSO-*d*₆) δ 17.5, 56.2, 56.6, 57.2, 104.1, 105.0, 107.3, 108.1, 116.0, 119.9, 120.8, 121.9, 123.7, 124.7, 133.5, 134.5, 145.4, 151.6, 152.7, 152.9, 156.3; Anal. calcd for C₂₄H₂₅NO₉S: C, 57.25, H, 5.00, N, 2.78; Found: C, 57.07. H, 5.17, N, 2.72.

*8-Methyl-3,4,10,11-tetramethoxydibenzo[a,g]quinolizinium acetosulfate* ***(2b)*:** prepared from **9b**; mp 267-269 °C dec (lit.¹⁴ mp 267-269 °C dec); IR (Nujol) 2762, 1702, 1642, 1595, 1546; ¹H NMR (DMSO-*d*₆) δ 3.40 (s, 3H), 3.43 (s, 3H), 4.00 (s, 3H), 4.09 (s, 3H), 4.13 (s, 6H), 7.77 (s, 1H), 7.85 (d, 2H, *J* = 8.1), 8.01 (d, 1H, *J* = 8.1), 8.75 (d, 1H, *J* = 8.0), 8.88 (d, 1H, *J* = 8.0), 9.67 (s, 1H); ¹³C NMR(DMSO-*d*₆) δ 17.7, 56.8, 56.9, 57.3, 61.6, 104.9, 105.5, 115.1, 116.3, 117.1, 119.5, 121.3, 122.3, 122.9, 126.9, 134.5, 135.5, 147.2, 153.1, 153.7, 156.9; Anal. calcd for C₂₄H₂₅NO₉S: C, 57.24; H, 5.00; N, 2.78. Found: C, 57.08; H, 5.35; N, 2.75.

*8-Methyl-3,4-methylenedioxy-10,11-dimethoxydibenzo[a,g]quinolizinium acetosulfate **(2c**):* prepared from **9c;** mp >270 °C dec; IR (Nujol) 3417, 1727, 1648, 1615, 1551; ¹H NMR (DMSO - *d*₆) 3.41 (s, 3H), 4.13 (s, 3H), 6.46 (s, 2H), 7.70 (d, 1H, *J* = 8.7), 7.76 (s, 1H), 7.81 (d, 1H, *J* = 8.0), 7.89 (s, 1H), 8.58 (d, 1H, *J*= 8.7), 8.84 (d, 1H, *J* = 8.0), 9.64 (s, 1H); Anal. calcd for C₂₃H₂₁NO₉S·2.25 H₂O: C, 52.32, H, 4.44, N, 2.65; Found: C, 52.21, H, 4.24, N, 2.68. HRMS calcd for C₂₁H₁₈NO₄: 384.1236; found 348.1237.

### Example III -General procedure for the synthesis of the phenylacetamides (5a-c).

A solution of 3,4-dimethoxyphenylacetyl chloride (4 mmol) in chloroform (6 mL) was added dropwise under nitrogen to a mixture of the appropriately substituted phenethylamine (4 mmol), chloroform (6 mL), and 2 M sodium carbonate (3 mL) at 0 °C with vigorous stirring. Stirring was continued at 0 °C until the reaction was complete (1-3 h). The reaction mixture was transferred to a separatory funnel using an additional 2 mL chloroform and 2 mL water. The organic phase was separated and the aqueous phase extracted with 5 mL chloroform. The combined chloroform extract was washed successively with 5 mL 0.1 N NaOH, 5 mL 0.1 N HCl and 5 mL saturated NaCl solution. The chloroform extract was dried (Na₂SO₄) and evaporated. The crude product was crystallized from methanol to give 95-100% yields of the pure amide as white needles.

*N-(3*,*4-dimethoxyphenylethyl)-2-(3,4-dimethoxyphenyl)acetamide* ***(5a)*:** prepared from 3,4-dimethoxyphenethylamine and 3,4-dimethoxyphenylacetyl chloride; mp 125 °C (lit.¹⁷ 123-125 °C); IR (KBr) 3327, 3064, 3007, 2916, 2840, 1642, 1608, 1591; ¹H NMR δ 2.67 (t, 2H), 3.40-3.47 (m, 4H), 3.83 (s, 6H), 3.86 (s, 3H), 3.88 (s, 3H), 5.30 (brs, 1H), 6.52-6.86 (m, 6H); ¹³C NMR δ 35.6, 41.2, 43.5, 56.3, 56.4, 70.5, 111.7, 112.2, 115.8, 121.1, 127.5, 127.9, 128.5, 129.1, 131.1, 131.6, 137.3, 148.1, 149.5, 158.5, 171.8.

*N-(2,3-dimethoxyphenylethyl)-2-(3,4-dimethoxyphenyl)acetamide* ***(5b)*** : prepared from 2,3-dimethoxyphenethylamine (Lindemann, *Helv. Chim. Acta.* 1949, 32, 69-75) and 3,4-dimethoxyphenylacetyl chloride; mp 80 °C (lit.²⁹ 79-80 °C); IR 3315, 2962, 2838, 1636, 1593, 1548; ¹H NMR δ 2.61 (t, 2H), 3.22-3.30 (m, 4H), 3.58 (s, 3H), 3.64 (s, 6H), 3.67 (s, 3H), 6.22 (brs, 1H), 6.44-6.79 (m, 6H); ¹³C NMR δ 30.0, 41.0, 43.7, 55.9, 56.2, 56.3, 60.9, 111.2, 111.9, 112.9, 122.0, 122.6, 124.4, 127.9, 133.1, 147.5, 148.5, 149.5, 153.1, 171.9.

*N-(2,3-methylenedioxyphenylethyl)-2-(3,4-dimethoxyphenyl)acetamide* ***(5c)*:** prepared from 2,3-methylenedioxyphenethylamine²⁸ and 3,4-dimethoxyphenylacetyl chloride; mp 124 °C ; IR (KBr) 3297, 3084, 2935, 1643, 1458; ¹H NMR δ 2.66 (t, 2H), 3.36-3.42 (m, 4H), 3.75 ( s, 3H), 3.79 (s, 3H), 5.73 (s, 2H), 5.81 (brs, 1H), 6.61-6.76 (m, 6H); ¹³C NMR δ 29.8, 39.7, 43.8, 56.4, 101.0, 107.5, 111.9, 112.9, 120.7, 122.0, 122.1, 123.3, 127.7, 146.0, 147.5, 148.7, 149.6, 171.8; Anal. calcd for C₁₉H₂₁NO₅: C, 66.46, H, 6.16, N, 4.08;
Found: C, 66.31, H, 6.16, N, 4.07.

### Example IV - General procedure for the synthesis of dihydroisoquinolines (6a-c).

The acetamide **(5a-c,** 2.47 mmol) was refluxed with phosphorus oxychloride (5.69 mmol) and dry toluene (10 mL), under nitrogen, for 20-60 min. The solvent was then carefully evaporated and the residue dissolved in ≈ 5 mL methanol. This solution was poured into ≈ 10-15 mL of cold water. After washing twice with 10 mL ether, 10 g of ice was added to the aqueous layer. While nitrogen was bubbled through the ice-chilled aqueous layer, the pH was adjusted to pH 10 with concentrated ammonium hydroxide. The aqueous layer was then saturated with sodium chloride and then extracted 5 times with 20 mL ether. The combined ether extract was dried over anhydrous potassium carbonate, filtered, and evaporated to give the dihydroisoquinolines, **6a-c.** As these compounds readily oxidize, exposure to air was avoided. The dihydroisoquinolines were used without further purification for the preparation of **7a-c, 8a-c,** and **9a-c.**

### Example V - General procedure for the synthesis of 5,6-dihydro-8-methyldibenzo[a,g]quinolizinium acetosulfates (7a-c).

Each of the dihydroisoquinolines **(6a-c,** 2.35 mmol) were dissolved in 1.0 mL of freshly distilled acetic anhydride. A procedure similar to that used for the synthesis of 8-methyldibenzo[a,g]quinolizinium acetosulfates **(2a-c)** was employed. The respective dihydroisoquinoline intermediates were used in place of the corresponding 1-benzylisoquinoline intermediates employed in the previous procedure. The products obtained were crystallized from boiling methanol to yield 85-90% of bright yellow crystalline product.

*5,6-Dihydro-8-methyl-2,3,10,11-tetramethoxydibenzo[a,g]quinolizinium acetosulfate (**7a**):* Prepared from **6a;** mp 278-279 °C (lit¹³ 277-279 °C); IR (KBr) 3450, 2946, 1725, 1611, 1567; ¹H NMR (DMSO-*d*₆) δ 3.18 (t, 2H), 3.23 (s, 3H), 3.40 (s, 3H), 3.89 (s, 3H), 3.94 (s, 3H), 4.08 (s, 6H), 4.75 (t, 2H), 7.13 (s, 1H), 7.63 (s, 2H), 7.80 (s, 1H), 8.74 (s, 1H); ¹³C NMR (DMSO-*d*₆) δ 17.8, 26.2, 49.8, 56.1, 56.3, 56.8, 57.3, 106.2, 106.4, 109.1, 110.9, 117.5, 120.0, 122.2, 128.6, 135.6, 139.0, 148.9, 151.6, 152.2, 155.4, 156.9, 167.5.

*5*,*6-Dihydro-8-methyl-3,4,10,11-tetramethoxydibenzo[a,g]quinolizinium acetosulfate* ***(7b):*** prepared from **6b;** mp 255-256 °C; IR (KBr) 3432, 2946, 1723, 1609, 1567, 1502, 1429; ¹H NMR (DMSO-*d*₆) δ 3.20 (t, 2H), 3.22 (s, 3H), 3.82 (s, 3H), 3.94 (s, 3H), 4.07 (s, 6H), 4.74 (t, 2H), 7.26 (d, 1H, *J*= 8.9), 7.67 (s, 1H), 7.78 (s, 1H), 7.87 (d, 1H, *J*= 8.9), 8.67 (s, 1H); ¹³C NMR (DMSO-*d*₆) δ 17.7, 21.0, 49.4, 56.3, 56.8, 57.3, 60.7, 106.3, 112.7, 117.6, 121.3, 122.3, 122.9, 128.9, 135.5, 138.9, 144.7, 152.3, 154.6, 155.3, 156.9, 167.6; Anal. calcd for C₂₄H₂₇NO₉S·1.25H₂O: C, 54.59, H, 5.63, N, 2.65; Found: C, 54.59, H, 5.60, N, 2.67; HRMS calcd for C₂₂H₂₄NO₄: 366.1705; found 366.1706.

*5,6-Dihydro-8-methyl-3,4-methylenedioxy-10,11-dimethoxydibenzo[a,g]quinolizinium acetosulfate (**7c**):* prepared from **6c;** mp >270 °C; IR (KBr) 3434, 2920, 1724, 1617; ¹H NMR (DMSO-*d*₆) δ 3.16 (t, 2H), 3.23 (s, 3H), 3.40 (s, 3H), 4.08 (s, 6H), 4.76 (t, 2H), 6.23 (s, 2H), 7.17 (d, 1H, *J* = 8.0), 7.67 (s, 1H), 7.70 (d, 1H, *J* = 8.0), 7.81 (s, 1H), 8.69 (s, 1H); ¹³C NMR (DMSO-*d*₆) 17.9, 20.8, 49.2, 56.8, 57.3, 102.7, 106.3, 106.5, 108.5, 116.2, 118.1, 121.2, 122.4, 122.7, 135.5, 139.0, 144.2, 149.6, 152.4, 155.6, 157.0, 167.6;; Anal. calcd for C₂₃H₂₃NO₉S·H₂O: C, 54.43, H, 4.96, N, 2.76; Found: C, 54.39, H, 4.96, N, 2.74; HRMS calcd for C₂₁H₂₀NO₄: 350.1392; found 350.1384.

### Example VI - General procedure for the synthesis of 5,6-dihydrodibenzo[a,g]quinolizinium chlorides (8a-c).

Phosphorus oxychloride (7.42 mmol) was added dropwise to chilled (0 °C) dimethylformamide under nitrogen. The mixture was stirred for 15 min at 0 °C. A solution of the respective dihydroisoquinolines (2.7 mmol) in 5.5 mL dimethylformamide was then added and allowed to stir at 0 °C for 1-2 h. The reaction mixture was then heated at 100 °C for 1-2 h. The reaction mixture was cooled to room temperature and poured into a mixture containing 20 g of ice and 10 mL 6 N HCl. The resulting precipitate was filtered and washed successively twice with 5 mL cold water and twice with 10 mL ether. The final product in each instance was recrystallized from methanol.

*5,6-Dihydro-2,3,10,11-tetramethoxydibenzo[a,g]quinolizinium chloride* ***(8a)*:** prepared from **6a;** mp 261-262 °C; IR (Nujol) 1664, 1660, 1564; ¹H NMR (CD₃OD) δ 3.35 (t, 3H), 3.91 (s, 3H), 4.02 (s,3H), 4.10 (s, 3H), 4.17 (s, 3H), 5.04 (t, 2H), 7.10 (s, 1H), 7.78 (s, 1H), 8.60 (s, 1H), 9.64 (s, 1H), 10.12 (s, 1H); ¹³C NMR (CD₃OD) δ 28.1, 56.4, 56.7, 56.9, 57.3, 57.8, 104.4, 106.6, 107.5, 108.1, 108.2,109.0, 110.2, 112.4, 112.5, 112.8, 115.4, 117.7,119.6, 130.4, 146.5; Anal. calcd for C₂₁H₂₂NO₄Cl·0.75H₂O: C, 62.84, H, 5.90, N, 3.49; Found: C, 62.79, H, 5.84, N, 3.46; HRMS calcd for C₂₁H₂₂NO₄: 352.1549; found 352.1549.

*5,6-Dihydro-3,4,10,11-tetramethoxydibenzo[a,g]quinolizinium chloride (8b):* prepared from **6b;** mp 252 °C; IR (Nujol) 3383, 1632, 1600, 1571; ¹H NMR (DMSO-*d*₆) δ 3.27 (t, 2H), 3.81 (s, 3H), 3.95 (s, 3H), 4.01 (s, 3H), 4.08 (s, 3H), 4.80 (t, 2H), 7.29 (d, 1H, *J* = 8.9), 7.68 (s, 1H), 7.73 (s, 1H), 7.96 (d, 1H, *J* = 8.9), 8.82 (s, 1H), 9.60 (s, 1H); ¹³C NMR (DMSO-*d*₆) δ 20.9, 54.4, 56.3, 56.6, 56.9, 60.6, 105.7, 106.7, 112.7, 118.4, 120.4, 122.4, 122.6, 129.1, 136.8, 138.5, 145.2, 145.7, 152.5, 154.9, 157.6; Anal. calcd for C₂₁H₂₂NO₄Cl·1.5H₂O: C, 60.79, H, 5.71, N, 3.38; Found: C, 60.79, H, 5.71, N, 3.38; HRMS calcd for C₂₀H₁₈NO₄: 336.1236; found 336.1244.

5, *6-Dihydro-3,4-methylenedioxy-10,11-dimethoxydibenzo[a,g]quinolizinium chloride* ***(8c)*:** prepared from **6c;** mp >280 °C; IR (Nujol) 2725, 1622, 1574; ¹H NMR (CD₃OD) δ 3.27 (t, 2H), 4.07 (s, 3H), 4.13 (s, 3H), 4.62 (t, 2H), 6.16 (s, 2H), 7.03 (d, 1H, *J*= 8.4), 7.60 (s, 1H), 7.63 (s, 1H), 7.75 (d, 1H, *J* = 8.4), 8.60 (s, 1H), 9.34 (s, 1H); ¹³C NMR (CD₃OD) δ 22.2, 56.0, 57.3, 57.7, 104.3, 106.6, 107.5, 109.6, 117.3, 120.1, 122.3, 122.9, 124.6, 139.2, 146.5, 146.6, 152.0, 155.0, 160.3 Anal. calcd for C₂₀H₁₈NO₄Cl·2H₂O: C, 58.89, H, 5.43, N, 3.43; Found: C, 58.74, H, 5.39, N, 3.45; HRMS calcd for C₂₀H₁₈NO₄: 336.1236; found 336.1244.

### Example VII - General procedure for the synthesis of 1-benzylisoquinolines (9a-c).

The appropriate dihydroisoquinoline (3.4 mmol) was refluxed at 220-230 °C with 0.3 g of 10% palladium on carbon in 5 mL tetralin (purged with nitrogen for 20 min prior to use) for 3-4 h. The reaction mixture was then allowed to cool to 180 °C and filtered under nitrogen using a Schlenk-type filtration unit (Aldrich Chemical Co.). After cooling to room temperature, the filtrate was chilled to 0 °C and hydrogen chloride (1.0 M in anhydrous ether) was added to adjust the pH of the mixture to pH 1.0. The hydrochloride salt of the 1-benzylisoquinoline precipitated. This precipitate was filtered, washed three times with 10 mL of anhydrous ether, and dried to give the respective 1-benzylisoquinoline hydrochloride salts in 90-100% yields. The dried hydrochloride salts were then dissolved in a minimum quantity of methanol (2-5 mL) to which was added 10 g of ice. Concentrated ammonium hydroxide was added to the ice-cooled aqueous solution to adjust the pH to 10. The aqueous layer was saturated with sodium chloride and then extracted with three 10 mL portions of chloroform. The combined chloroform extract was dried (Na₂SO₄). and evaporated to give **9a-c.**

*5,6-Dimethoxy-1-(3,4-dimethoxybenzyl)isoquinoline hydrochloride* ***(9b)*:** prepared from **6b;** mp 206-208 °C (lit.¹⁴ 206-208 °C); IR (Nujol) 2676, 1630, 1625, 1588; ¹H NMR (CD₃OD) δ 3.76 (s, 3H), 3.81 (s, 3H), 4.03 (s, 3H), 4.15 (s, 3H), 4.87 (s, 2H), 6.86 (t, 2H), 7.07 (s, 1H), 7.92 (d, 1H, *J* = 1), 8.31 (d, 2H, *J* = 5.7), 8.55 (d, 1H, *J* = 1); ¹³C NMR (CD₃OD) δ 38.1, 56.9, 57.1, 58.1, 62.5, 113.7, 114.2, 119.2, 120.8, 122.5, 122.8, 127.7, 129.1, 131.2, 135.9, 143.5, 150.5, 151.3, 158.5, 160.2.

*5,6-Methylenedioxy-1-(3,4-dimethoxybenzyl)isoquinoline (**9c**):* prepared from **6c;** mp 111 °C ; IR (KBr) 3429, 3064, 3003, 2930; ¹H NMR (CD₃OD) δ 2.93 (s, 3H), 4.03 (s, 3H), 4.04 (s, 3H), 6.01 (s, 2H), 6.91 (d, 1H, J = 8.8) 7.07 (s, 1H), 7.25 (s,1H), 7.60 (m, 2H), 7.68 (s, 1H); ¹³C NMR δ (CD₃OD) 23.2, 56.5, 76.9, 77.4, 78.2, 101.6, 104.4, 106.0, 107.8, 109.9, 114.1, 121.0, 122.5, 134.0, 135.2, 148.1, 149.2, 150.1, 153.1, 156.2; Anal. calcd for C₁₉H₁₇O₄: C, 70.58, H, 5.30, N, 4.33; Found: C, 70.85. H, 5.51, N, 4.31.

### Example VIII - General procedure for the synthesis of dibenzo[a,g]quinolizinium chlorides (10a-c).

The procedure employed was similar to that used in the synthesis of 5,6-dihydrodibenzo[a,g]quinolizinium chlorides, **8a-c.** The 1-benzylisoquinoline intermediates were substituted for corresponding dihydroisoquinoline intermediates in the previous procedure. The products obtained were crystallized from a 1:1 mixture of glacial acetic acid and 6 N HCl to give yields ranging from 92-98% of **10a-c** as yellow crystalline products.

*2,3,10,11-Tetramethoxydibenzo[a,g]quinolizinium chloride* ***(10a)*:** prepared from **9a;** mp, IR, ¹H NMR, ¹³C NMR were as previously reported.¹²

*3,4,10,11-Tetramethoxydibenzo[a,g]quinolizinium chloride* ***(10b)*:** prepared from **9b;** mp 223-225 °C dec; IR (KBr) 3394, 2947, 2843, 1626, 1598, 1561, 1503, 1433; ¹H NMR (using a coaxial tube with **9b** dissolved in trifluoroacetic acid in the outer tube and deuterium oxide in the inner tube) δ 4.51 (s, 3H), 4.55 (s, 6H), 4.61 (s, 3H), 7.94 (s, 1H), 7.96 (s, 1H), 8.06 (d, 1H, *J* = 9.2), 8.46 (d, 1H, *J*= 7.3), 8.78 (d, 1H, *J* = 7.3), 9.01 (d, 1H, J = 9.2), 9.56 (s, 1H), 9.83 (s, 1H); ¹³C NMR (using a coaxial tube with the **9b** dissolved in trifluoroacetic acid in the outer tube and deuterium oxide in the inner tube) δ 58.4, 58.8, 59.1, 64.7, 107.0, 107.2, 118.9, 119.2, 119.7, 121.6, 125.0, 126.8, 127.2, 132.0, 139.4, 139.5, 140.0, 145.1, 156.6, 157.9, 161.1; Anal. calcd for C₂₁H₂₀NO₄Cl·1.25 H₂O: C, 61.76, H, 5.55, N, 3.43; Found: C, 60.67, H, 5.37, N, 3.43; HRMS calcd for C₂₁H₂₀NO₄: 350.1392; found 350.1392.

*3,4-Methylenedioxy-10,11-dimethoxydibenzo[a,g]quinolizinium chloride* ***(10c)*:** prepared from **9c;** mp >270 °C dec; IR (KBr) 3414, 3015, 2928, 1620, 1564, 1488; ¹H NMR (using a coaxial tube with the compound dissolved in trifluoroacetic acid in the outer tube and deuterium oxide in the inner tube) δ 4.55 (s, 3H), 4.60 (s, 3H), 6.67 (s, 2H), 7.81 (d, 1H, *J* = 8.5), 7.92 (s, 2H), 8.18 (d, 1H, *J*= 7.7), 8.68 (t, 2H), 9.47 (s, 1H), 9.77 (s, 1H); ¹³C NMR (using a coaxial tube with the compound dissolved in trifluoroacetic acid in the outer tube and deuterium oxide in the inner tube) δ 58.8, 59.1, 106.7, 107.0, 107.1, 115.2, 115.7, 118.4, 119.8, 121.7, 121.8, 126.6, 131.1, 139.5, 139.9, 140.3, 147.1, 153.6, 156.4, 161.1; Anal. calcd for C₂₀H₁₆NO₄Cl·1.75H₂O: C, 59.86, H, 4.90, N, 3.49; Found: C, 59.72, H, 4.93, N, 3.48; HRMS calcd for C₂₀H₁₆NO₄: 334.1079; found 334.1075.

### Example IX - 3,4,3',4'-Tetramethoxydesoxybenzoin (11a).

Powdered anhydrous aluminum chloride (0.78 g) was slowly added to a stirred mixture of 1,2-dimethoxybenzene (0.64 g, 4.66 mmol) and 3,4-dimethoxyphenylacetyl chloride (1.0 g, 4.66 mmol) in 10 mL freshly distilled dry dichloromethane. An exothermic reaction occurred. The orange solution became brown as the mixture refluxed. The reaction mixture was heated to reflux for an additional 2 h and then allowed to cool to room temperature. The cooled solution was poured into a mixture containing of 5 g crushed ice and 5.5 mL 7.5 N HCl. The organic phase was separated and the aqueous phase was extracted three times with 10 mL dichloromethane. The combined dichloromethane extract was dried (Na₂SO₄), filtered, and evaporated to give a white solid, which was crystallized from ethanol to give pure white needles of **11a** in 98% yield; mp 105-107 °C (lit.³⁰ mp 104-106 °C); ¹H NMR δ 3.85 (s, 6H), 3.91 (s, 3H), 3.94 (s, 3H), 4.18 (s, 2H), 6.81 (m, 2H), 6.91 (m, 2H), 7.56 (d, 1H, *J* = 2.0), 7.66 (dd, 1H, *J* = 8.4, 2.0); ¹³C NMR δ 45.2, 56.3, 56.4, 56.5, 110.4, 111.1, 111.8, 112.8, 121.3, 121.9, 123.9, 127.9, 130.2, 148.4, 149.5, 153.8, 197.0.

### Example X - 3,4-Dimethoxy-3',4'-methylenedioxydesoxybenzoin (11b).

A solution of 3,4-dimethoxyphenylacetyl chloride (3.25 g, 15 mmol) in 15 mL freshly distilled dry dichloromethane was added dropwise to a stirred mixture of 1,3-benzodioxole (1.83 g, 15 mmol) and tin(IV) chloride (4.6 g, 17.6 mmol) in 15 mL dichloromethane at -10 °C. The mixture was then allowed to rise to room temperature and was stirred for an additional 2 h. The reaction mixture was then poured into 25 mL 6 N HCl and stirred for 16 h. The organic phase was then separated and the aqueous phase was extracted three times with 20 mL aliquots of dichloromethane. The combined dichloromethane extract was washed successively with 20 mL of 0.1 N NaOH and 20 mL distilled water. The dichloromethane extract was then dried (Na₂SO₄), filtered, and evaporated to give a cream colored solid. The crude product was crystallized from ethanol to give 3.0 g of white needles of **11b** in 66% yield; mp 110-111 °C; IR (Nujol) 2725, 1675, 1589, 1519; ¹H NMR δ 3.80 (s, 3H), 3.81 (s, 3H), 4.09 (s, 2H), 5.96 (s, 2H), 6.76 (m, 4H), 7.42 (d, 1H, *J* = 1.5), 7.58 (dd, 1H, *J* = 6.4, 1.5); ¹³C NMR δ 45.3, 56.3, 102.3, 108.3, 108.7, 111.8, 112.7, 112.9, 121.9, 125.4, 127.7, 131.8, 148.4, 148.6, 149.4, 152.2, 196.4; Anal. calcd for C₁₇H₁₆O₅: C, 67.99, H, 5.37; Found: C, 67.98. H, 5.32.

### Example XI - General procedure for the synthesis of 1-methyl-3-phenylisoquinolines (12a, 12b).

Anhydrous phosphorus pentoxide (4 mmol) was added in three portions to a 10 mL of a solution (100 mM) of the respective desoxybenzoins in acetonitrile. The reaction mixture was stirred under nitrogen for 18-20 h. The reaction mixture was quenched by addition of 10 mL water. The resulting suspension was neutralized with 10% NaOH and extracted with three 10 mL portions of dichloromethane. The combined organic phase was dried (Na₂SO₄), filtered, and evaporated to give a cream colored residue. This residue was chromatographed on silica gel using a 95:5 mixture of dichloromethane and ethyl acetate, respectively, as eluent to give the corresponding 1-methyl-3-phenylisoquinolines, **12a** and **12b,** in 75-85% yield.

*6,7-Dimethoxy-1-methyl-3-(3,4-dimethoxyphenyl)isoquinoline* ***(12a):*** prepared from **11a;** mp 168-169 °C (lit.³¹ 168-170 °C); IR (Nujol) 2725, 1621, 1573, 1508; ¹H NMR δ 2.95 (s, 3H), 3.93 (s, 3H), 4.02 (s, 9H), 6.96 (d, 1H, *J* = 8.4), 7.08 (s, 1H), 7.25 (s, 1H), 7.60 (dd, 1H, *J* = 8.4, 2.0), 7.71 (s, 2H); ¹³C NMR δ 23.2, 56.4, 104.3, 105.9, 110.4, 111.7, 113.9, 119.5, 122.4, 133.6, 133.9, 149.2, 149.6, 149.7, 150.0, 153.1, 156.2.

*6*,*7-Dimethoxy-1-methyl-3-(3, 4-methylenedioxyphenyl)isoquinoline* ***(12b)*:** prepared from **11b;** mp 174 °C; IR (Nujol) 2727, 1622, 1573, 1504; ¹H NMR δ 2.93 (s, 3H), 4.03 (s, 3H), 4.04 (s, 3H), 6.00 (s, 2H), 6.91 (1H, d, *J*= 8.8), 7.07 (s, 1H), 7.25 (s, 1H), 7.60 (m, 2H), 7.67 (s, 1H); ¹³C NMR δ 23.2, 56.5, 101.6, 104.4, 106.0, 107.8, 108.9, 114.1, 121.0, 122.5, 133.9, 135.2, 148.1, 148.6, 149.2, 150.1, 153.1, 156.2; Anal. calcd for C₁₉H₁₇NO₄: C, 70.58, H, 5.30, N, 4.33; Found: C, 70.55. H, 5.28, N, 4.30.

### Example XII - 6,7-Dihydroxy-1-methyl-3-(3,4-dihydroxyphenyl)isoquinoline (13).

Compound **12a** (250 mg, 0.74 mmol) was dissolved in 5 mL dry chloroform and chilled to -50 °C using a mixture of dry ice and acetone. To this mixture was added dropwise 7.4 mL of a 1.0 M solution of boron tribromide in dichloromethane. The reaction mixture was allowed to come to room temperature over a period of 4 h. The precipitate was filtered and washed twice with 2 mL portions of ether. This crude product was recrystallized from ethanol to give **13** in 98% yield as white needles; mp >280 °C dec; IR (Nujol) 3326, 1602, 1531; ¹H NMR (CD₃OD) δ 3.08 (s, 3H), 6.98 (d, 1H, *J* = 8.1), 7.19 (dd, 1H, *J* = 8.1, 1.9), 7.24 (s, 1H), 7.37 (s, 1H), 7.61 (s, 1H), 7.94 (s, 1H); ¹³C NMR (CD₃OD) δ 17.8, 109.6, 110.6, 116.1, 117.3, 119.6, 121.2, 122.8, 125.6, 138.4, 142.5, 147.7, 149.5, 152.0, 154.3, 158.3; HRMS calcd for C₁₆H₁₃NO₄: 283.0844; found 283.0839.

### Example XIII - General procedure for the synthesis of 1,2-dimethyl-3-phenylisoquinolinium derivatives (14a, 14b, 15).

Each of the respective 1-methyl-3-phenylisoquinoline derivatives (0.67 mmol) was added to 1 mL of dimethyl sulfate. This reaction mixture was then heated at 100 °C for 20-60 min and then allowed to cool to room temperature. Anhydrous ether (8 mL) was added to the cooled reaction mixture and the resulting suspension stirred for 5 min. The precipitate was filtered and recrystallized from methanol to give 95-100% of the corresponding N-methylisoquinolinium salts.

*6,7-Dimethoxy-1, 2-dimethyl-3-(3, 4-dimethoxyphenyl)isoquinolinium methosulfate (**14a**):* prepared from **12a;** mp 224-226 °C; IR (Nujol) 3508, 1640, 1613, 1548, 1506; ¹H NMR (CD₃OD) δ 3.21 (s, 3H), 3.67 (s, 3H), 3.94 (s, 3H), 3.98 (s, 3H), 4.11 (s, 3H), 4.12 (s, 1H), 7.20 (d, 1H, *J* = 8.6), 7.48 (m, 2H), 7.62 (s, 1H), 7.66 (s, 1H), 8.24 (s, 1H); ¹³C NMR (CD₃OD) δ 18.0, 56.9, 57.1, 57.3, 57.6, 106.0, 107.5, 112.8, 113.5, 120.7, 122.7, 123.2, 126.4, 139.1, 151.5, 170.4, 170.9, 171.3; Anal. calcd for C₂₂H₂₇NO₈S·1.5H₂O: C, 53.65, H, 5.83, N, 2.84; Found: C, 53.35. H, 5.52, N, 2.91.

6,*7-Dimethoxy-1,2-dimethyl-3-(3, 4-methylenedioxyphenyl)isoquinolinium methosulfate (**14b**):* prepared from **12b;** mp 235-237 °C; IR (Nujol) 3479, 1612, 1568; ¹H NMR (DMSO-*d*₆) δ 3.22 (s, 3H), 3.38 (s, 3H), 4.06 (s, 6H), 6.20 (s, 2H), 7.14 (m, 2H), 7.23 (m, 1H), 7.70 (s, 1H), 7.86 (s, 1H), 8.09 ( s, 1H); ¹³C NMR (DMSO-*d*₆) δ 18.2, 43.5, 56.8, 56.9, 102.2, 106.2, 106.3, 109.1, 110.0, 122.9, 123.2, 124.1, 127.7, 134.7, 144.9, 147.9, 149.0, 152.5, 156.9, 157.1; Anal. calcd for C₂₁H₂₃NO₈S: C, 53.11, H, 5.15, N, 3.11; Found: C, 53.03. H, 5.19, N, 2.95.

*6,7-Dihydroxy-1,2-dimethyl-3-(3, 4-dihydroxyphenyl)isoquinolinium methosulfate (**15**):* prepared from **13**; mp 118-120 °C; IR (KBr) 3249, 1614, 1528, 1454; ¹H NMR (CD₃OD) δ 3.08 (s, 3H), 3.68 (s, 3H), 6.98 (d, 1H, *J* = 8.2), 7.21 (m, 2H), 7.37 (s, 1H), 7.61 (s, 1H), 7.94 (s, 1H); ¹³C NMR (CD₃OD) δ 17.8, 109.6, 110.6, 116.1, 117.3, 119.6, 121.2, 122.8, 125.6, 138.4, 142.6, 147.7, 149.5, 152.0, 154.3, 158.3; Anal. calcd for C₁₈H₁₉NO₈S: C, 52.81, H, 4.68, N, 3.42; Found: C, 52.79, H, 4.65, N, 3.40.

### Example XIV - Materials.

The plasmid pET11a and the *E*. *coli* strain BL21(DE3) used for enzyme expression were purchased from Novagen. IPTG was purchased from Sigma. The ECL system used for the Western blotting analysis of bacterial lysates was from Amersham (UK). All the restriction enzymes and Vent polymerase were from New England Biolabs. Mammalian topoisomerase II was isolated from calf thymus glands according to the published procedure (Halligan et al., *J. Biol. Chem. 260*:2475-2482 (1985)). The single copy yeast plasmids YCpGAL1 expressing various topoisomerase I genes in JN2-134 yeast strain were a kind gift of Dr. M-A. Bjornsti (Thomas Jefferson University, Philadelphia, PA). All bacterial and yeast media were from Difco (Detroit, MI), while cell culture media were purchased from Gibco-BRL (Gaithersburg, MD).

### Example XV - Topoisomerase I expression in E. coli.

To obtain large quantity of human topoisomerase I, the human topoisomerase I cDNA was cloned into the pET-11a vector, in which transcription of the cDNA is under the control of the inducible T7 promoter (Studier et al., Methods in Enzymol., Vol. 185:60-89, San Diego: Academic Press (1990)). Briefly, a 3.4 kb DNA fragment containing the entire coding sequence of human topoisomerase I and approximately 1 kb of untranslated region downstream of the stop codon was isolated from the plasmid YCpGAL1-hTOP1 (Bjornsti et al., *Cancer Res. 49:*6318-6323 (1989)) by cutting at the BamHI and EcoRI sites. The vector pET-11a was cut with the same restriction enzymes, dephosphorylated and ligated to the insert in the proper reading-frame downstream of the vector cloning site. The ligation mixture was used to transform *E*. *coli,* the correct clone pET1B was isolated (see figure 2A for the map) and its identity confirmed by restriction mapping. Since the translational start in pET is positioned at an upstream NdeI site, the expressed topoisomerase I has a 15 amino acid fusion at its N-terminus. pET1B was then transformed into *E. coli* BL21(DE3), and, upon induction with 0.4 mM IPTG for 1 hour, the bacterial lysate was analyzed by 10 % SDS-PAGE. Expression was confirmed by Western blotting using rabbit antibodies against human topoisomerase I. Isolation of the expressed protein was done by a simple procedure. Briefly, *E*. *coli* cells were lysed by repeated sonic bursts. The sonic extract was made in 1 M NaCl and 6% polyethylene glycol (PEG) to remove nucleic acids. The PEG supernatant was chromatographed directly on a hydroxyapatite column. Expressed human DNA topoisomerase I was eluted at the 0.6 M potassium phosphate step. The eluted enzyme was dialyzed against 50% glycerol, 30 mM potassium phosphate (pH 7.0), 1 mM dithiothreitol (DTT) and 0.1 mM EDTA and stored at -20°C. The relaxation activity of the purified enzyme had a specific activity about 2 orders of magnitude lower than the calf thymus topoisomerase I.

### Example XVI - Expression of camptothecin-resistant (CPT-K5) topoisomerase I in E. coli.

Two complementary oligonucleotides containing the point mutation CAG (Asp533)->CGG (Gly) responsible for the resistance phenotype in CPT-K5 (19), were synthesized and engineered in the topo I coding sequence using the sequential PCR method (Current Protocols in Molecular Biology, *In:* Ausubel et al. (eds.), Vol. 1, pp. 8.5.7. Boston:Wiley Interscience (1991)). The two oligonucleotides are 5'-CTTCCTCGGGAAGGGCTCCATCAGATAC-3' (primer X1), and 5'-GTATCTGATGGAGCCCTTCCCGAGGAAG-3' (primer X2), where the underlined sequence represents the mutated codon. Each oligonucleotide was used in separate PCR reactions to amplify two DNA segments adjacent to the mutation site, using the oligonucleotides
5'-ACTGTGATCCTAGGG-3' ("A") and
5'-CTTCATCGACAAGCTTGCTCTGAG-3' ("H") as the relative primer pairs for X1 and X2, respectively. "A" and "H" are complementary to the human topo I sequence around the unique restriction sites AvrII and HindIII. After the first round of PCR, the two amplified products X1-H and X2-A were denatured and annealed by their 15 base-pair complementary sequence, due to the overlap of the oligonucleotides X1 and X2. This short stretch of double-stranded DNA segment was then extended by Vent polymerase at 72°C for 2 minutes to the supposedly 748 base pairs full length product A-H. The two external primers "A" and "H" were then used to amplify the full length DNA fragment containing the mutated topo I fragment. The amplified mutant topoisomerase I cDNA was then digested with AvrII and HindIII, and cloned into pET1B by replacing the corresponding AvrII/HindIII fragment in the topoisomerase I cDNA sequence. The plasmid pET1B-CPTK5, which contained the mutant CPT-K5 topoisomerase I cDNA instead of the wildtype human topoisomerase I cDNA, was transformed into *E*. *coli* BL21(DE3) for expression. Upon induction with IPTG, the protein in the lysates was confirmed by Western blotting. The CPT-K5 topoisomerase I was then purified from the bacterial lysate as described for the wildtype enzyme.

### Example XVII - Topo I and topo II cleavage assay.

Cleavage assays for the recombinant topoisomerases I and calf thymus topoisomerases I and II were done as described (Liu et al., *J. Biol. Chem. 258:*15365-15370 (1983)). The plasmid YEpG DNA used for the cleavage assays was prepared and labeled at its 3'-end using the published procedures.

### Example XVIII - Yeast cytotoxicity assay.

It has been established that yeast can survive when topoisomerase I function is obliterated, and that the topoisomerase I poisons only kill cells having a functional topoisomerase I (Bjornsti et al. *Cancer Res. 49:*6318-6323 (1989)). Thus, comparison of the relative extent of growth of each of the test strains in the presence of various drugs with control plates minus drug shows 1) whether the drug has any cytotoxic effects on yeast, 2) whether the cytotoxicity is topo I specific and 3) whether there is any differential specificity of the drug for yeast compared with human topo I.

The topoisomerase I-specific *in vivo* cytotoxicity assay was adapted from Knab et al. (Knab et al., *J. Biol. Chem. 268:*22322-22330 (1993)). In this system, various topo I genes cloned into the single copy yeast plasmid vector, YCpGAL1 (Knab et al., *J. Biol. Chem. 268:*22322-22330 (1993)), are expressed under the control of the GAL1 promoter in the JN2-134 strain of S. cerevisiae (MATa, rad52::LEU2, trp1, ade2-1,his7, ura3-52, isel, top1-1, leu2) (Bjornsti et al., *Cancer Res. 49:*6318-6323 (1989)). The topo I constructs in the vector are, respectively, the wild-type yeast topo I (YCpGAL-ScTOP1), a non-functional yeast topo I where the active site tyrosine-727 is mutated to a phenylalanine (YCpGAL1-Sctop1Y727F) (Knab et al., *J. Biol. Chem. 268*:22322-22330 (1993)), and the wild type human topoisomerase I (YCpGAL-hTOP1) (Bjornsti et al., *Cancer Res. 49:*6318-6323 (1989)). To qualitatively test the cytotoxicity and the topo I specificity of the drugs, yeast cells containing the specific plasmid were serially diluted (5-fold) and were grown in dropout medium supplemented with uracil and 2% galactose In addition, the plates contained: A: Control, no drug in the plate; B: Camptothecin (CPT), 0.5 µM; C: Coralyne, 1 µM ; D: Methylenedioxy-dihydro-demethyl-coralyne (MDD-Coralyne), 1 µM, and E: Nitidine, 1 µM. The plates were grown for 3 days at 30°C to assess the lethal effect of the different compounds on the various topoisomerase I enzymes expressed in *S. cerevisiae*.and the drug being tested.

### Example XIX - Cytotoxicity assay.

The IC₅₀ of the drugs tested were determined by the MTT-microtiter plate tetrazolinium cytotoxicity assay (MTA) (Mosmann, T., *J. Immunol*. *Methods 65:*55-63 (1983); Denizot et al., *J. Immunol*. *Methods 89*:271-277 (1986)). Human lymphoblast RPMI 8402 cells and their camptothecin-resistant CPT-K5 cells (Andoh et al., *Proc. Natl. Acad. Sci., USA 84:*5565-5569 (1987)) were kindly provided by Dr. Toshiwo Andoh (Aichi Cancer Center Research Institute, Nagoya, Japan). The cell lines A2780 and its camptothecin-resistant derivative CPT-2000 were a generous gift of Dr. Jaulang Hwang (Institute of Molecular Biology, Academia sinica, Taiwan). Cells (2000 cells/well, seeded in 200 ml growth medium) were grown in suspension at 37°C in 5% CO₂ and maintained by regular passage in RPMI medium supplemented with 10% heat inactivated fetal bovine serum, L-glutamine (2 mM), penicillin (100 U/ml), and streptomycin (0.1 mg/ml). The cells were exposed continuously for 4 days to different drug concentrations, and assayed at the end of the fourth day. Each concentration and the no drug control were repeated at least twice in 6 replica wells. The results were plotted and the IC50 then measured. The drug sensitive human epidermoid carcinoma KB 3-1 cell line and its vinblastine-selected multidrug-resistant variant KB-V1 cells (Akiyama et al., *Genetics 11:*117-126 (1985)) were kindly provided by Dr. Michael Gottesmann (National Cancer Institute). They were grown as monolayer cultures at 37°C in 5% CO2 and maintained by regular passage in Dulbecco's minimal essential medium supplemented with 10% heat inactivated fetal bovine serum. KB-V1 cells were maintained in the presence of 1 mg/ml vinblastine.

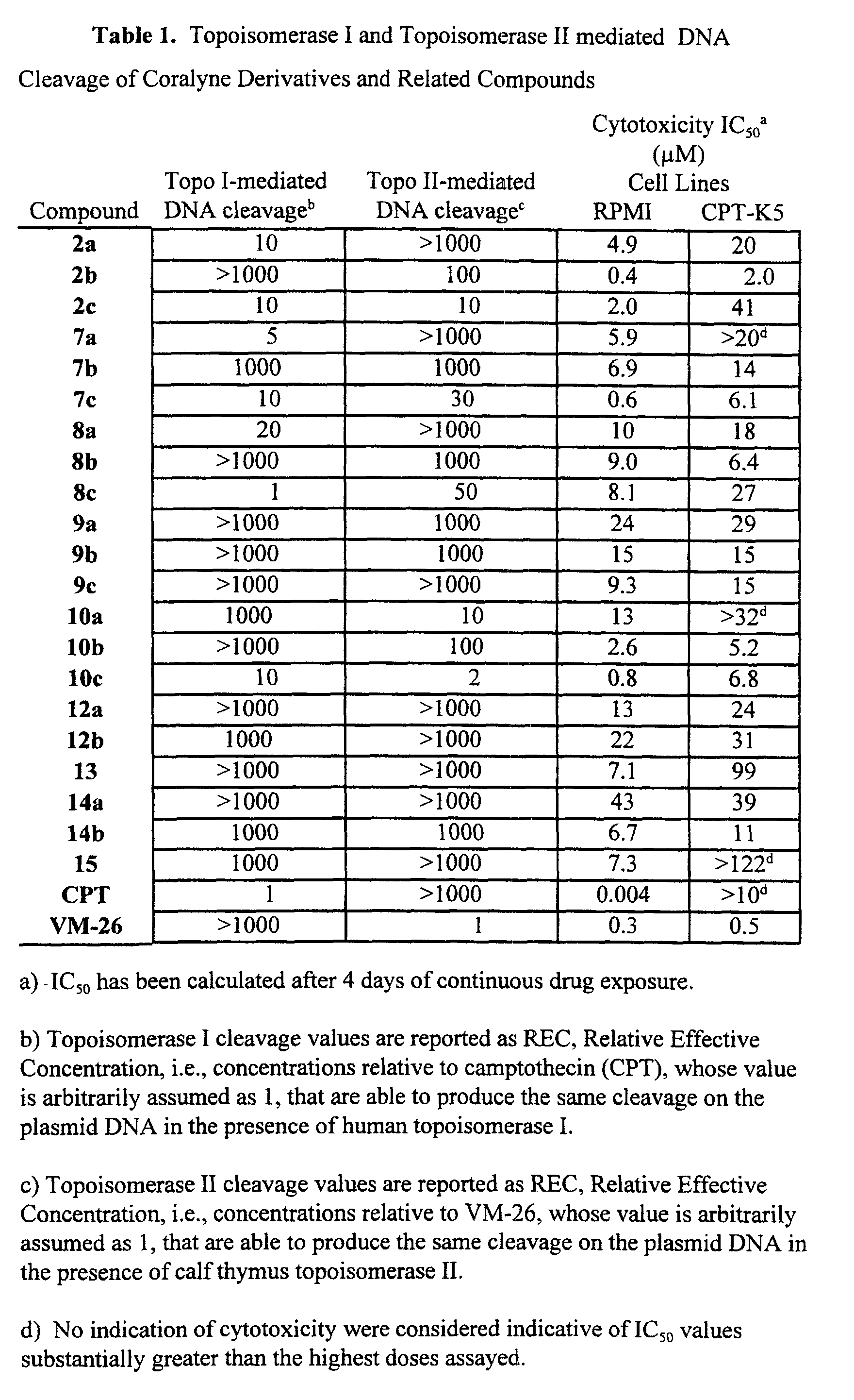

The coralyne analog **7c** was tested for its effectiveness against glioblastoma cells. The assay used was similar to that described above for the RPMI-8402 cell line. The results are shown in table 2 below which compares the effect of Compound 7c against the leukemia cell line (RPMI 8402), epidermal carncinoma (KB3-1) and glioblastoma (SF-268):

**Table 2**

| | Cytotoxicity (ug/ml) | | |
|---|---|---|---|
| Compound | RPMI 8402 | KB3-1 | SF-268 |
| coralyne | 3.0 | 0.9 | 0.1 |
| Compound **7c** | 0.3 | 0.06 | 0.008 |
| doxorubicine | 0.8 | 0.1 | 0.8 |
| camptothecin | 0.003 | 0.004 | 0.005 |

### Example XX - Results

In order to establish whether human DNA topoisomerase I is the cytotoxic target of coralyne and its derivatives, human topoisomerase I cDNA were expressed in a T7 expression system. The expressed human DNA topoisomerase I was purified by a single chromatographic step. The recombinant human DNA topoisomerase I was used to evaluate the activity of coralyne and its derivatives. The recombinant human DNA topoisomerase I and topoisomerase I purified from calf thymus glands have comparable cleavage activity and produce similar cleavage patterns in the presence of camptothecin and coralyne.

The recombinant human DNA topoisomerase I induced extensive DNA breaks on YEpG DNA in the presence of either coralyne or camptothecin. These DNA breaks were shown to reflect the formation of topoisomerase I cleavable complexes by the following criteria: (1) They represent single-strand breaks since no breaks were observable if the reactions were not denatured prior to loading onto the neutral agarose gel; (2) the breaks were shown to be protein-linked and reversible upon heating to 65°C, a hallmark of topoisomerase I cleavable complexes. The cleavage pattern induced by coralyne is similar if not identical to that of camptothecin, suggesting that the two drugs may share a similar mode of action against topoisomerase I.

To test whether coralyne is a dual poison, coralyne's activity against calf thymus DNA topoisomerase II was evaluated. It was determined that coralyne has essentially no activity against purified calf thymus DNA topoisomerase II. Both nitidine and VM-26 were used as controls for comparison. Nitidine, like VM-26 but unlike coralyne, is a highly potent topoisomerase II poison.

The fact that coralyne is a rather specific topoisomerase I poison allows evaluation of the role of topoisomerase I as the cytotoxic target of coralyne in cells. Yeast *top1* deletion strains expressing human DNA topoisomerase I have been successfully applied to demonstrate the role of human DNA topoisomerase I as the sole cytotoxic target of camptothecin in yeast cells. Although, coralyne exhibited no cytotoxicity against yeast strains expressing human topoisomerase I or non-functional yeast topoisomerase I, some of the coralyne derivatives were highly cytotoxic against yeast cells expressing human topoisomerase I. MDD-coralyne is highly cytotoxic against yeast cells expressing human DNA topoisomerase I but not cytotoxic against yeast cells expressing either functional or non-functional yeast topoisomerase I. This result suggests that the cytotoxic target of MDD-coralyne is human topoisomerase I in the yeast system. It was surprising that yeast cells expressing functional yeast topoisomerase I are resistant to coralyne, as they are quite sensitive to camptothecin. Since nitidine is structurally similar to MDD-coralyne, its activity in the yeast system was also evaluated. Nitidine, like MDD-coralyne, is highly cytotoxic against yeast cells expressing human topoisomerase I but not cytotoxic against yeast cells expressing either functional or non-functional yeast topoisomerase I. This result again supports the notion that human topoisomerase I is the cytotoxic target of nitidine and that yeast topoisomerase I is resistant to nitidine.

To evaluate the possible role of topoisomerase I as the cytotoxic target in human cells, coralyne derivatives were tested against two pairs of camptothecin-resistant cell lines (RPMI8402/CPT-K5 and A2780/CPT2000). In both pairs of cell lines, the resistance to camptothecin has been attributed to mutations within the structural genes of human topoisomerase I leading to camptothecin resistant human topoisomerase I (Tamura et al., *Nucl. Acids Res. 19*: 69-75 (1991)). As shown in Table 1, whereas the resistance index for camptothecin is in the range of 1000 to 10,000, the resistance index for coralyne derivatives and nitidine varied between 1 to 9.

In view of the similar cleavage pattern produced by camptothecin and coralyne derivatives (also nitidine), the lack of significant cross-resistance of the camptothecin-resistant cell lines to coralyne derivatives and nitidine was surprising. It may indicate that these drugs have additional cytotoxic target(s). Alternatively, camptothecin-resistant topoisomerase I in the resistant cells simply does not confer resistance to coralyne derivatives and nitidine due to non-overlapping receptor sites. In order to distinguish between these two possibilities, camptothecin-resistant topoisomerase I was isolated from CPT-K5 cells and tested for resistance/sensitivity to coralyne derivatives and nitidine. Unfortunately, the CPT-K5 topoisomerase I purified from CPT-K5 cells was not active enough to give conclusive results. To overcome this problem, *in vitro* site-directed mutagenesis was performed to create an A to G transitional mutation at nucleotide position 1809 of the human topoisomerase I cDNA. The mutation which causes a Asp to Gly (a.a. # 533) change in the amino acid sequence of the protein has been shown to be responsible for camptothecin resistance (Tamura et al., *Nucl. Acids Res. 19:* 69-75 (1991)). The mutation was engineered into human topoisomerase I cDNA on pET1B. The resulting plasmid, pET1B/CPT-K5, was used for overexpression of CPT-K5 topoisomerase I. The overexpressed recombinant CPT-K5 topoisomerase I was partially purified and shown to have about the same specific activity as the wildtype human topoisomerase I expressed and purified in a similar manner. The recombinant CPT-K5 topoisomerase I was shown to be highly resistant (over 1000 fold) to camptothecin. However, the recombinant CPT-K5 topoisomerase I was only marginally resistant to nitidine (less than 10 fold) and moderately resistant to D-coralyne (about 30-fold). Surprisingly, the recombinant CPT-K5 topoisomerase I is highly resistant to coralyne as evidenced by the cleavage assay.

As shown in Table 2, the compound **7c** has exceptional cytotoxicity against glioblastoma cells, and is more cytotoxic against this CNS tumor cell than the leukemia or epidermal carcinoma cell lines. Its effectiveness against SF-268 cells suggests that selective uptake of this compound may be occurring.

These studies demonstrate that coralyne analogs exhibit enhanced activity as topoisomerase I and/or topoisomerase II poisons. These data suggest that similarly substituted analogs of nitidine could exhibit similar selectivity with regard to inhibition of mammalian topoisomerase I or II.

## Claims

1. A compound of the formula: 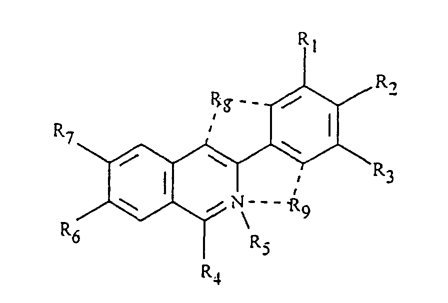 wherein
R₁, R₂, R₆ and R₇ are independently H, OH, or (C₁-C₈)alkoxy;
R₃ is OH or (C₁-C₈)alkoxy; R₂ and
R₃ together are -OCH₂O-; R₁ and R₂ together are -OCH₂O-; or R₆ and R₇ together are -OCH₂O-;
R₄ is H or (C₁-C₈)alkyl;
R₅ is H, (C₁-C₈)alkyl or is absent; and
R₈ and R₉ are independently -CH=CH-, -(CH₂)₂ or are absent;
provided that
when R₈ is -CH=CH- or -(CH)₂-, R₉ is absent and R₃ is H; and
when R₉ is -CH=CH- or -(CH₂)₂, R₁ or R₂ is H, and R₅ and R₈ are absent;
or a pharmaceutically acceptable salt thereof; for use in inhibiting cancer cell growth.

2. A compound for use according to Claim 1 wherein the cancer cell is located in the central nervous system.

3. A compound for use according to Claim 1 wherein the cancer is a leukemia or melanoma.

4. A compound for use according to Claim 1 wherein the cancer is a solid tumor.

5. A compound for use according to Claim 1 wherein the cancer is a breast, lung, colon, or ovarian tumor.

6. A compound of the formula: 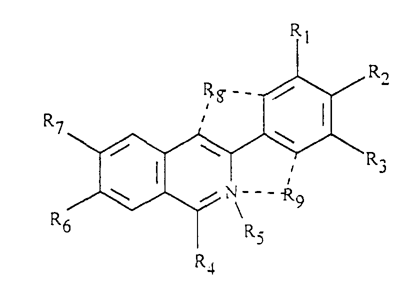 wherein
R₁, R₂, R₆ and R₇ are independently H, OH, or (C₁-C₈)alkoxy; R₁ and R₂ together are -OCH₂O-; or R₆ and R₇ together are -OCH₂O-;
R₃ is H;
R₄ is H or (C₁-C₈)alkyl;
R₅ is H, (C₁-C₈)alkyl or is absent;
R₈ -CH=CH-; and
R₉ is absent; or a pharmaceutically acceptable salt thereof.

7. The compound of claim 6 wherein R₆ and R₇ are -OCH₃.

8. The compound of claim 7 wherein R₁ is -OCH₃.

9. The compound of claim 7 wherein R₂ is -OCH₃.

10. A compound of the formula: 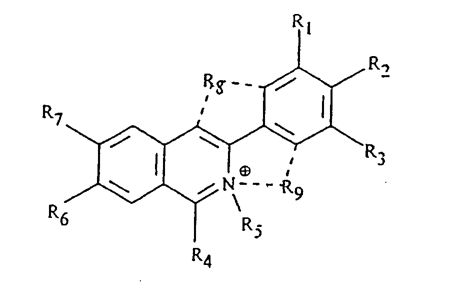 wherein
R₁, R₂, R₆ and R₇ are independently H, OH, or (C₁-C₈)alkoxy;
R₃ is OH or (C₁-C₈)alkoxy; R₂ and
R₃ together are -OCH₂O-; R₁ and R₂ together are -OCH₂O-; or R₆ and R₇ together are -OCH₂O-;
R₄ is H or (C₁-C₈)alkyl;
R₅ is absent;
R₈ is absent; and
R₉ is -CH=CH- or -(CH₂)₂;
provided that R₁ or R₂ is H;
or a pharmaceutically acceptable salt thereof.

11. The compound of claim 10 wherein R₆ and R₇ are -OCH₃.

12. The compound of claim 11 wherein R₄ is CH₃.

13. The compound of claim 12 wherein R₁ is H, R₂ and R₃ are -OCH₃.

14. The compound of claim 12 wherein R₁ is H, and R₂ and R₃ are -OCH₂O-.

15. The compound of claim 11 wherein R₄ is H.

16. The compound of claim 15 wherein R₁ is H, R₂ and R₃ are -OCH₃.

17. The compound of claim 15 wherein R₁ is H, and R₂ and R₃ are -OCH₂O-.

18. A compound of the formula: 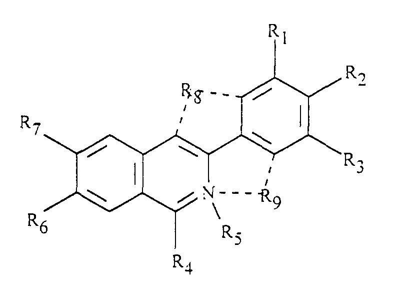 wherein
R₁, R₂, and R₃ are independently H, OH, or (C₁-C₈)alkoxy; R₂ and R₃ together are -OCH₂O-; or R₁ and R₂ together are -OCH₂O-;
R₄ is H or (C₁-C₈)alkyl;
R₅ is H, (C₁-C₈)alkyl or is absent;
R₆ and R₇ are each OH; and
R₈ and R₉ are each absent;
or a pharmaceutically acceptable salt thereof.

19. The compound of claim 18 wherein R₄ is CH₃.

20. The compound of claim 19 wherein R₅ is CH₃.

21. The compound of claim 20 wherein R₁ is H, and R₂ and R₃ are each OH.

22. A method of using a compound of the formula: 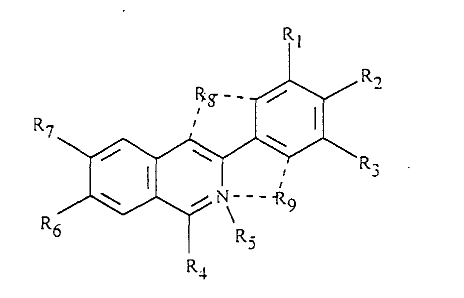 wherein
R₁, R₂, R₃, R₆ and R₇ are independently H, OH, or (C₁-C₈)alkoxy; R₂ and
R₃ together are -OCH₂O-; R₁ and R₂ together are -OCH₂O-; or R₆ and R₇ together are -OCH₂O-;
R₄ is H or (C₁-C₈)alkyl;
R₅ is H, (C₁-C₈)alkyl or is absent; and
R₈ and R₉ are independently -CH=CH-, -(CH₂)₂ or are absent;
provided that
when R₈ is -CH=CH- or -(CH)₂-, R₉ is absent and R₃ is H; and
when R₉ is -CH=CH- or -(CH₂)₂, R₁ or R₂ is H, and R₅ and R₈ are absent;
or a pharmaceutically acceptable salt thereof, to prepare a medicament effective to inhibit tumor cell growth in a mammal with cancer

23. A pharmaceutical composition comprising an effective amount of a compound of the formula: 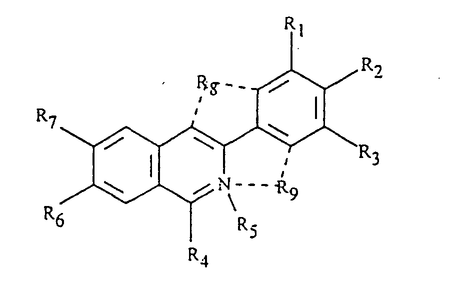 wherein
R₁, R₂, R₆ and R₇ are independently H, OH, or (C₁-C₈)alkoxy;
R₃ is OH or (C₁-C₈)alkoxy; R₂ and
R₃ together are -OCH₂O-; R₁ and R₂ together are -OCH₂O-; or R₆ and R₇ together are -OCH₂O-;
R₄ is H or (C₁-C₈)alkyl;
R₅ is H, (C₁-C₈)alkyl or is absent; and
R₈ and R₉ are independently -CH=CH-, -(CH₂)₂ or are absent;
provided that
when R₈ is -CH=CH- or -(CH)₂-, R₉ is absent and R₃ is H; and
when R₉ is -CH=CH- or -(CH₂)₂, R₁ or R₂ is H, and R₅ and R₈ are absent:
or a pharmaceutically acceptable salt thereof; in combination with a pharmaceutically acceptable carrier.

24. The compound 8-methyl-3,4,10,11-tetramethoxydibenzo[a,g]quinolizinium acetosulfate; 8-methyl-3,4-methylenedioxy-10,11-dimethoxydibenzo[a,g]-quinolizinium acetosulfate; 5.6-dihydro-8-methyl-3,4,10,11-tetramethoxy-dibenzo[a,g]quinolizinium acetosulfate; 5,6-dihydro-8-methyl-3,4-methylenedioxy-10,11-dimethoxydibenzo[a.g]quinolizinium acetosulfate; 5,6-dihydro-3,4,10,11-tetramethoxydibenzo[a,g]quinolizinium chloride; 5,6-dihydro-3,4-methylenedioxy-10,11-dimethoxydibenzo[a,g]quinolizinium chloride; 3,4,10,11-tetramethoxy-dibenzo[a,g]quinolizinium chloride; or 3,4-methylenedioxy-10,11-dimethoxy-dibenzo[a,g]quinolizinium chloride; or a pharmaceutically acceptable salt thereof.

25. The compound 6,7-dimethoxy-1-methyl-3-(3,4-methylenedioxyphenyl)isoquinoline; 6,7-dihydroxy-1-methyl-3-(3,4-dihydroxyphenyl)isoquinoline; 6,7-dimethoxy-1,2-dimethyl-3-(3,4-methylenedioxyphenyl)isoquinolinium methosulfate; or 6,7-dihydroxy-1,2-dimethyl-3-(3,4-dihydroxyphenyl)-isoquinolinium methosulfate; or a pharmaceutically acceptable salt thereof.

26. A compound of the formula: 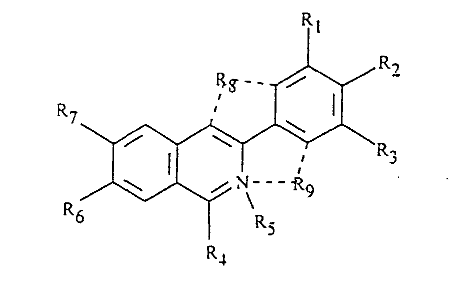 wherein
R₁, R₂, R₆ and R₇ are independently H, OH, or (C₁-C₈)alkoxy; R₁ and R₂ together are -OCH₂O-; or R₆ and R₇ together are -OCH₂O-; R₃ is H;
R₄ is H or (C₁-C₈)alkyl;
R₅ is H, (C₁-C₈)alkyl or is absent;
R₈ is -(CH₂)₂-; and R₉ is absent; or a pharmaceutically acceptable salt thereof.

27. A compound of the formula: 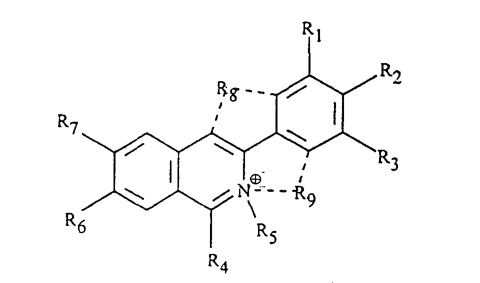 wherein
R₁, R₂, R₃, R₆ and R₇ are independently H, OH, or (C₁-C₈)alkoxy; R₂ and R₃ together are -OCH₂O-; or R₆ and R₇ together are -OCH₂O-;
R₄ is H or (C₁-C₈)alkyl;
R₅ is absent;
R₈ is absent; and
R₉ is -CH=CH-;
provided that R₁ or R₂ is H;
or a pharmaceutically acceptable salt thereof.

28. A compound of the formula: 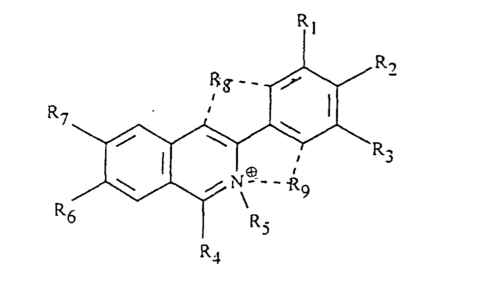 wherein
R₁, R₂, R₃, R₆ and R₇ are independently H, OH, or (C₁-C₈)alkoxy; R₂ and R₃ together are -OCH₂O-; or R₆ and R₇ together are -OCH₂O-; or
R₄ is H or (C₁-C₈)alkyl;
R₅ is absent;
R₈ is absent; and
R₉ is -(CH₂)₂- ;
provided that R₁ or R₂ is H;
or a pharmaceutically acceptable salt thereof.

29. A compound of the formula: 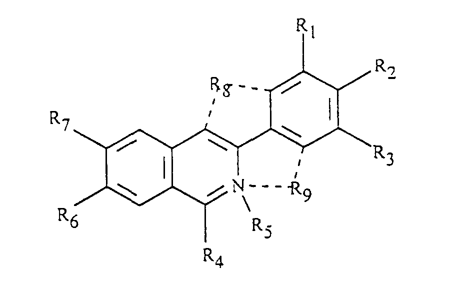 wherein
R₃, R₆ and R₇ are independently H, OH, or (C₁-C₈)alkoxy; or R₆ and R₇ together are -OCH₂O-;
R₁ and R₂ together are -OCH₂O-;
R₄ is H or (C₁-C₈)alkyl;
R₅ is H, (C₁-C₈)alkyl or is absent; and
R₈ and R₉ are each absent;
or a pharmaceutically acceptable salt thereof.

30. A compound of the formula: 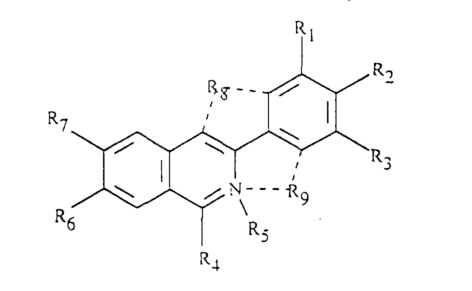 wherein
R₁, R₆, and R₇ are independently H, OH, or (C₁-C₈)alkoxy; or R₆ and R₇ together are -OCH₂O-;
R₂ and R₃ together are -OCH₂O-;
R₄ is H or (C₁-C₈)alkyl;
R₅ is H, (C₁-C₈)alkyl or is absent; and
R₈ and R₉ are each absent;
or a pharmaceutically acceptable salt thereof.

31. A compound of the formula: 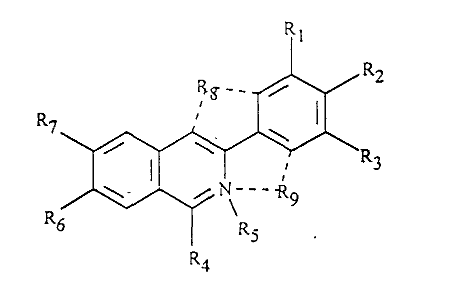 wherein
R₁, R₂, and R₃ are independently H, OH, or (C₁-C₈)alkoxy; R₂ and R₃ together are -OCH₂O-; or R₁ and R₂ together are -OCH₂O-;
R₄ is H or (C₁-C₈)alkyl;
R₅ is H, (C₁-C₈)alkyl or is absent;
R₆ and R₇ together are -OCH₂O-; and
R₈ and R₉ are each absent;
or a pharmaceutically acceptable salt thereof.

## Patentansprüche

1. Verbindung der Formel: 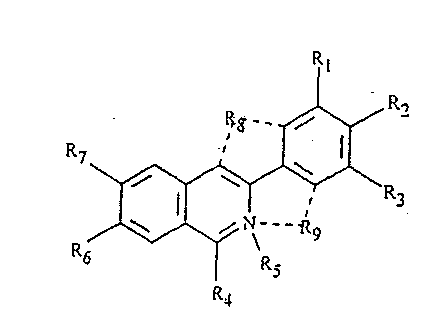 worin
R₁ , R₂ , R₆ und R₇ unabhängig voneinander H, OH oder (C₁-C₈)Alkoxy sind, R₃ OH oder (C₁-C₈)Alkoxy ist, R₂ und R₃ zusammen -OCH₂O- sind, R₁ und R₂ zusammen -OCH₂O- sind oder R₆ und R₇ zusammen -OCH₂O- sind,
R₄ H oder (C₁-C₈)Alkyl ist,
R₅ H, (C₁-C₈)Alkyl oder nicht vorhanden ist, und
R₈ und R₉ unabhängig voneinander -CH=CH-, - (CH₂)₂ oder nicht vorhanden sind,
mit der Maßgabe, daß,
wenn R₈ -CH=CH- oder -(CH)₂- ist, R₉ nicht vorhanden und R₃ H ist, und
wenn R₉ -CH=CH- oder -(CH₂)₂ ist, R₁ oder R₂ H ist und R₅ und R₈ nicht vorhanden sind,
oder ein pharmazeutisch geeignetes Salz davon zur Verwendung bei der Inhibition von Krebszellwachstum.

2. Verbindung zur Verwendung nach Anspruch 1, wobei sich die Krebszelle im Zentralnervensystem befindet.

3. Verbindung zur Verwendung nach Anspruch 1, wobei der Krebs Leukämie oder ein Melanom ist.

4. Verbindung zur Verwendung nach Anspruch 1, wobei der Krebs ein fester Tumor ist.

5. Verbindung zur Verwendung nach Anspruch 1, wobei der Krebs ein Brust-, Lungen-, Darm- oder Eierstocktumor ist.

6. Verbindung der Formel 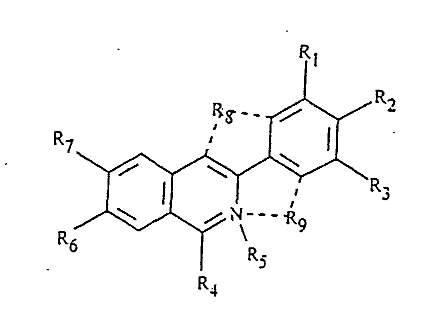 worin
R₁ , R₂, R₆ und R₇ unabhängig voneinander H, OH oder (C₁-C₈)Alkoxy sind, R₁ und R₂ zusammen -OCH₂O- sind oder R₆ und R₇ zusammen -OCH₂O- sind,
R₃ H ist,
R₄ H oder (C₁-C₈)Alkyl ist,
R₅ H, (C₁-C₈)Alkyl oder nicht vorhanden ist,
R₈ -CH=CH- ist und
R₉ nicht vorhanden ist,
oder ein pharmazeutisch geeignetes Salz davon.

7. Verbindung nach Anspruch 6, worin R₆ und R₇ -OCH₃ sind.

8. Verbindung nach Anspruch 7, worin R₁ -OCH₃ ist.

9. Verbindung nach Anspruch 7, worin R₂ -OCH₃ ist.

10. Verbindung der Formel 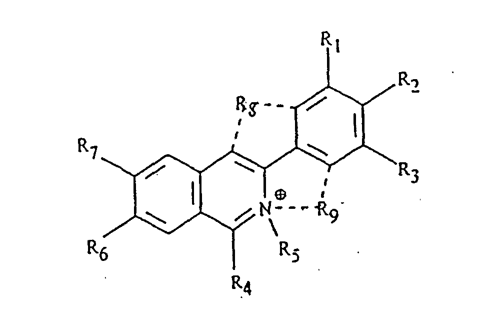 worin
R₁, R₂ , R₆ und R₇ unabhängig voneinander H, OH oder (C₁-C₈)Alkoxy sind, R₃ OH oder (C₁-C₈)Alkoxy ist, R₂ und R₃ zusammen -OCH₂O- sind, R₁ und R₂ zusammen -OCH₂O- sind oder R₆ und R₇ zusammen -OCH₂O- sind,
R₄ H oder (C₁-C₈)Alkyl ist,
R₅ nicht vorhanden ist,
R₈ nicht vorhanden ist und
R₉ -CH=CH- oder -(CH₂)₂ ist,
mit der Maßgabe, daß R₁ oder R₂ H ist,
oder ein pharmazeutisch geeignetes Salz davon.

11. Verbindung nach Anspruch 10, worin R₆ und R₇ -OCH₃ sind.

12. Verbindung nach Anspruch 11, worin R₄ -CH₃ ist.

13. Verbindung nach Anspruch 12, worin R₁ H ist und R₂ und R₃ -OCH₃ sind.

14. Verbindung nach Anspruch 12, worin R₁ H ist und R₂ und R₃ -OCH₂O- sind.

15. Verbindung nach Anspruch 11, worin R₄ H ist.

16. Verbindung nach Anspruch 15, worin R₁ H ist und R₂ und R₃ -OCH₃ sind.

17. Verbindung nach Anspruch 15, worin R₁ H ist und R₂ und R₃ -OCH₂O- sind.

18. Verbindung der Formel 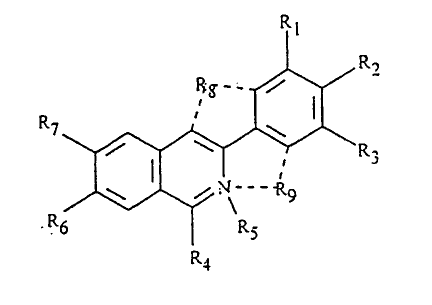 worin
R₁ , R₂ und R₃ unabhängig voneinander H, OH oder (C₁-C₈)Alkoxy sind, R₂ und R₃ zusammen -OCH₂O- sind oder R₁ und R₂ zusammen -OCH₂O- sind,
R₄ H oder (C₁-C₈)Alkyl ist,
R₅ H, (C₁-C₈)Alkyl oder nicht vorhanden ist,
R₆ und R₇ jeweils -OH sind und
R₈ und R₉ jeweils nicht vorhanden sind,
oder ein pharmazeutisch geeignetes Salz davon.

19. Verbindung nach Anspruch 18, worin R₄ -CH₃ ist.

20. Verbindung nach Anspruch 19, worin R₅ -CH₃ ist.

21. Verbindung nach Anspruch 20, worin R₁ H ist und R₂ und R₃ jeweils -OH sind.

22. Verfahren zur Verwendung einer Verbindung der Formel 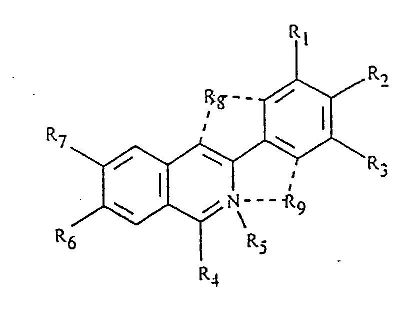 worin
R₁ , R₂ , R₃ , R₆ und R₇ unabhängig voneinander H, OH oder (C₁-C₈)Alkoxy sind, R₂ und R₃ zusammen -OCH₂O- sind, R₁ und R₂ zusammen -OCH₂O- sind oder R₆ und R₇ zusammen -OCH₂O- sind,
R₄ H oder (C₁-C₈)Alkyl ist,
R₅ H, (C₁-C₈)Alkyl oder nicht vorhanden ist, und
R₈ und R₉ unabhängig voneinander -CH=CH-, -(CH₂)₂ oder nicht vorhanden sind,
mit der Maßgabe, daß,
wenn R₈ -CH=CH- oder -(CH)₂- ist, R₉ nicht vorhanden und R₃ H ist, und
wenn R₉ -CH=CH- oder -(CH₂)₂ ist, R₁ oder R₂ H ist und R₅ und R₈ nicht vorhanden sind,
oder eines pharmazeutisch geeigneten Salzes davon zur Herstellung eines Arzneimittels, welches wirksam ist, das Tumorzellwachstum in einem von Krebs befallenen Säuger zu inhibieren.

23. Pharmazeutische Zusammensetzung, welche eine wirksame Menge einer Verbindung der Formel 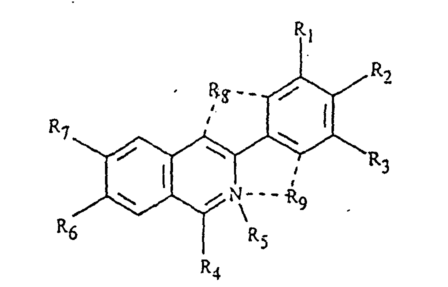 worin
R₁ , R₂ , R₆ und R₇ unabhängig voneinander H, OH oder (C₁-C₈)Alkoxy sind, R₃ OH oder (C₁-C₈)Alkoxy ist, R₂ und R₃ zusammen -OCH₂O- sind, R₁ und R₂ zusammen -OCH₂O- sind oder R₆ und R₇ zusammen -OCH₂O- sind,
R₄ H oder (C₁-C₈)Alkyl ist,
R₅ H, (C₁-C₈)Alkyl oder nicht vorhanden ist, und
R₈ und R₉ unabhängig voneinander -CH=CH-, -(CH₂)₂ oder nicht vorhanden sind,
mit der Maßgabe, daß,
wenn R₈ -CH=CH- oder -(CH)₂- ist, R₉ nicht vorhanden und R₃ H ist, und
wenn R₉ -CH=CH- oder -(CH₂)₂ ist, R₁ oder R₂ H ist und R₅ und R₈ nicht vorhanden sind,
oder eines pharmazeutisch geeigneten Salzes davon in Kombination mit einem pharmazeutisch geeigneten Träger enthält.

24. Verbindung 8-Methyl-3,4,10,11-Tetramethoxydibenzo[a,g]-chinoliziniumacetosulfat, 8-Methyl-3,4-methylendioxy-10, 11-dimethoxydibenzo[a,g]chinoliziniumacetosulfat, 5,6-Dihydro-8-methyl-3,4,10,11-tetramethoxydibenzo[a,g]chinoliziniumacetosulfat, 5,6-Dihydro-8-methyl-3,4-methylendioxy-10,11-dimethoxydibenzo[a,g]chinoliziniumacetosulfat, 5,6-Dihydro-3,4,10,11-tetramethoxydibenzo[a,g]chinoliziniumchlorid, 5,6-Dihydro-3,4-methylendioxy-10,11-dimethoxydibenzo[a,g]-chinoliziniumchlorid, 3,4,10,11-Tetramethoxydibenzo[a,g]-chinoliziniumchlorid oder 3,4-Methylendioxy-10,11-dimethoxydibenzo[a,g]chinoliziniumchlorid oder ein pharmazeutisch geeignetes Salz davon.

25. Verbindung 6,7-Dimethoxy-1-methyl-3-(3,4-methylendioxyphenyl)isochinolin, 6,7-Dihydroxy-1-methyl-3-(3,4-dihydroxyphenyl)isochinolin, 6,7-Dimethoxy-1,2-dimethyl-3-(3,4-methylendioxyphenyl)isochinoliniummethosulfat oder 6,7-Dihydroxy-1,2-dimethyl-3-(3,4-dihydroxyphenyl)isochinoliniummethosulfat oder ein pharmazeutisch geeignetes Salz davon.

26. Verbindung der Formel 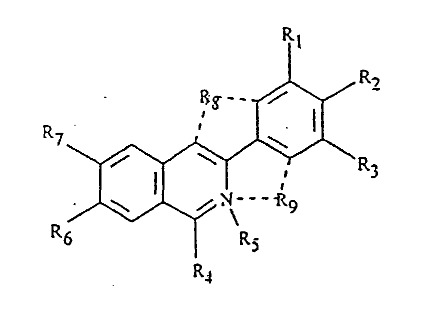 worin
R₁ , R₂ , R₆ und R₇ unabhängig voneinander H, OH oder (C₁-C₈)Alkoxy sind, R₁ und R₂ zusammen -OCH₂O- sind oder R₆ und R₇ zusammen -OCH₂O- sind, und R₃ H ist,
R₄ H oder (C₁-C₈)Alkyl ist,
R₅ H, (C₁-C₈)Alkyl oder nicht vorhanden ist,
R₈ - (CH₂)₂ ist und
R₉ nicht vorhanden ist,
oder ein pharmazeutisch geeignetes Salz davon.

27. Verbindung der Formel 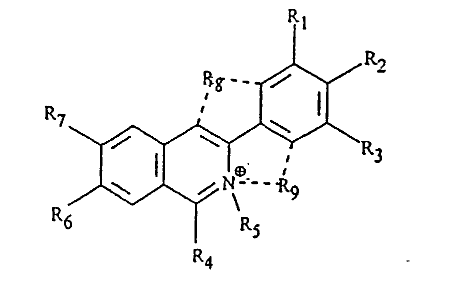 worin
R₁ , R₂ , R₃ , R₆ und R₇ unabhängig voneinander H, OH oder (C₁-C₈)Alkoxy sind, R₂ und R₃ zusammen -OCH₂O- sind oder R₆ und R₇ zusammen -OCH₂O- sind,
R₄ H oder (C₁-C₈)Alkyl ist,
R₅ nicht vorhanden ist,
R₈ nicht vorhanden ist und
R₉ -CH=CH- ist,
mit der Maßgabe, daß R₁ oder R₂ H ist,
oder ein pharmazeutisch geeignetes Salz davon.

28. Verbindung der Formel 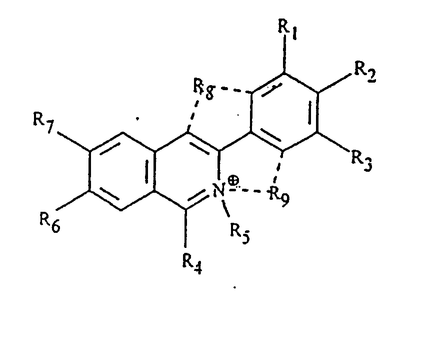 worin
R₁ , R₂ , R₃ , R₆ und R₇ unabhängig voneinander H, OH oder (C₁-C₈)Alkoxy sind, R₂ und R₃ zusammen -OCH₂O- sind oder R₆ und R₇ zusammen -OCH₂O- sind,
R₄ H oder (C₁-C₈)Alkyl ist,
R₅ nicht vorhanden ist,
R₈ nicht vorhanden ist und
R₉ -(CH₂)₂- ist,
mit der Maßgabe, daß R₁ oder R₂ H ist,
oder ein pharmazeutisch geeignetes Salz davon.

29. Verbindung der Formel 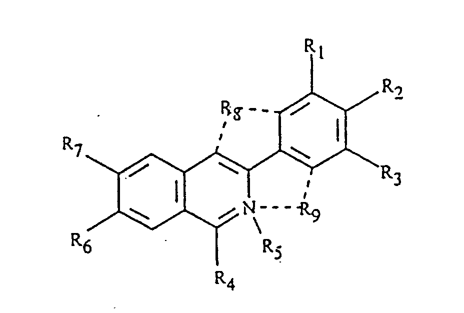 worin
R₃ , R₆ und R₇ unabhängig voneinander H, OH oder (C₁-C₈)Alkoxy sind oder R₆ und R₇ zusammen -OCH₂O- sind,
R₁ und R₂ zusammen -OCH₂O- sind,
R₄ H oder (C₁-C₈)Alkyl ist,
R₅ H, (C₁-C₈)Alkyl oder nicht vorhanden ist und
R₈ und R₉ nicht vorhanden sind,
oder ein pharmazeutisch geeignetes Salz davon.

30. Verbindung der Formel 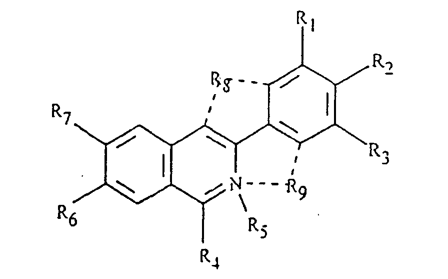 worin
R₁ , R₆ und R₇ unabhängig voneinander H, OH oder (C₁-C₈)Alkoxy sind oder R₆ und R₇ zusammen -OCH₂O- sind,
R₂ und R₃ zusammen -OCH₂O- sind,
R₄ H oder (C₁-C₈)Alkyl ist,
R₅ H, (C₁-C₈)Alkyl oder nicht vorhanden ist und
R₈ und R₉ nicht vorhanden sind,
oder ein pharmazeutisch geeignetes Salz davon.

31. Verbindung der Formel 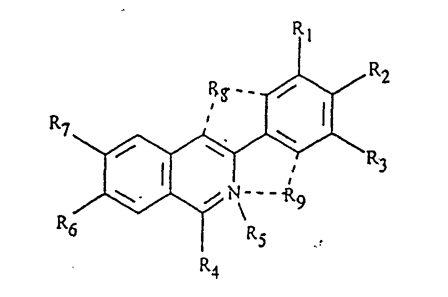 worin
R₁ , R₂ , R₃ unabhängig voneinander H, OH oder (C₁-C₈)Alkoxy sind, R₂ und R₃ zusammen -OCH₂O- sind oder R₁ und R₂ zusammen -OCH₂O- sind,
R₄ H oder (C₁-C₈)Alkyl ist,
R₅ H, (C₁-C₈)Alkyl oder nicht vorhanden ist,
R₆ und R₇ zusammen -OCH₂O- sind und
R₈ und R₉ jeweils nicht vorhanden sind,
oder ein pharmazeutisch geeignetes Salz davon.

## Revendications

1. Composé de formule 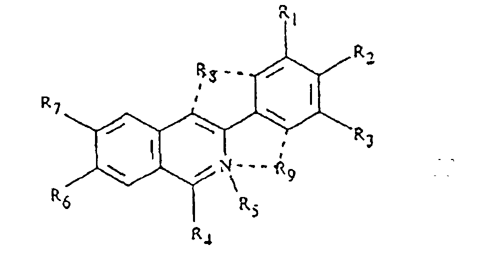 dans laquelle
R₁, R₂, R₆ et R₇ sont chacun indépendamment H, OH ou un groupe alcoxy en C₁-C₈; R₃ est OH ou un groupe alcoxy en C₁-C₈; R₂ et R₃ forment ensemble -OCH₂O-; R₁ et R₂ forment ensemble -OCH₂O- ; ou R₆ et R₇ forment ensemble -OCH₂O- ;
R₄ est H ou un groupe alkyle en C₁-C₈,
R₅ est H, un groupe alkyle en C₁-C₈, ou est absent ; et
R₈ et R₉ sont indépendamment -CH=CH-, -(CH₂)₂-, ou sont absents ;
à la condition que quand R₈ est -CH=CH- ou -(CH)₂-, R₉ soit absent et R₃ soit H ; et
quand R₉ est -CH=CH- ou -(CH₂)₂-, R₁ ou R₂ soit H, et R₅ et R₈ soient absents ;
ou un de ses sels acceptables d'un point de vue pharmaceutique ; pour utilisation dans l'inhibition de la prolifération des cellules cancéreuses.

2. Composé pour utilisation selon la revendication 1, dans lequel la cellule cancéreuse est située dans le système nerveux central.

3. Composé pour utilisation selon la revendication 1, dans lequel le cancer est une leucémie ou un mélanome.

4. Composé pour utilisation selon la revendication 1, dans lequel le cancer est une tumeur solide.

5. Composé pour utilisation selon la revendication 1, dans lequel le cancer est une tumeur du sein, des poumons, du colon ou des ovaires.

6. Composé de formule 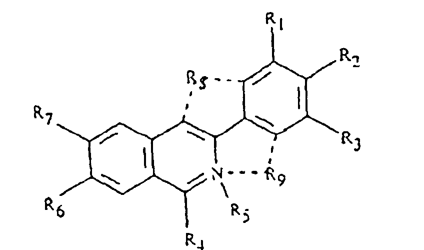 dans laquelle R₁, R₂, R₆ et R₇ sont indépendamment H, OH ou un groupe alcoxy en C₁-C₈ ; R₁ et R₂ forment ensemble -OCH₂O- ; ou R₆ et R₇ forment ensemble -OCH₂O- ;
R₃ est H;
R₄ est H ou un groupe alkyle en C₁-C₈ ;
R₅ est H, un groupe alkyle en C₁-C₈, ou est absent ;
R₈ est -CH=CH- ; et
R₉ est absent ; ou l'un de ses sels acceptables d'un point de vue pharmaceutique.

7. Composé selon la revendication 6, dans lequel R₆ et R₇ représentent chacun -OCH₃.

8. Composé selon la revendication 7, dans lequel R₁ est -OCH₃.

9. Composé selon la revendication 7, dans lequel R₂ est -OCH₃.

10. Composé de formule 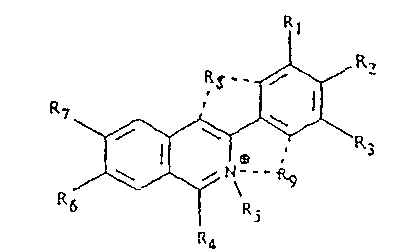 dans laquelle
R₁, R₂, R₆ et R₇ sont indépendamment H, OH ou un groupe alcoxy en C₁-C₈; R₃ est OH ou un groupe alcoxy en C₁-C₈; R₂ et R₃ forment ensemble -OCH₂O- ; R₁ et R₃ forment ensemble -OCH₂O- ; ou R₆ et R₇ forment ensemble -OCH₂O- ;
R₄ est H ou un groupe alkyle en C₁-C₈;
R₅ est absent ;
R₈ est absent ; et
R₉ est -CH=CH ou -(CH₂)₂;
à la condition que R₁ ou R₂ soit H ;
ou l'un de ses sels acceptables d'un point de vue pharmaceutique.

11. Composé selon la revendication 10, dans lequel R₆ et R₇ sont -OCH₃.

12. Composé selon la revendication 11, dans lequel R₄ est CH₃.

13. Composé selon la revendication 12, dans lequel R₁ est H, R₂ et R₃ sont -OCH₃.

14. Composé selon la revendication 12, dans lequel R₁ est H, et R₂ et R₃ sont -OCH₂O-.

15. Composé selon la revendication 11, dans lequel R₄ est H.

16. Composé selon la revendication 15, dans lequel R₁ est H, R₂ et R₃ sont -OCH₃.

17. Composé selon la revendication 15, dans lequel R₁ est H, et R₂ et R₃ sont -OCH₂O-.

18. Composé de formule 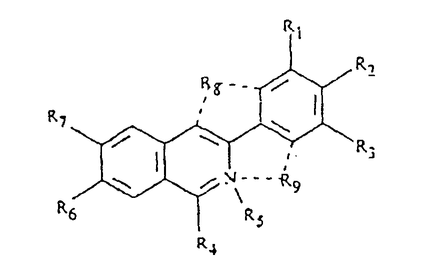 dans laquelle
R₁, R₂ et R₃ sont indépendamment H, OH ou un groupe alcoxy en C₁-C₈ ; R₂ et R₃ forment ensemble -OCH₂O-; ou R₁ et R₂ forment ensemble -OCH₂O- ;
R₄ est H ou un groupe alkyle en C₁-C₈;
R₅ est H, un groupe alkyle en C₁-C₈, ou est absent ;
R₆ et R₇ sont chacun OH ; et
R₈ et R₉ sont chacun absents ;
ou l'un de ses sels acceptables d'un point de vue pharmaceutique.

19. Composé selon la revendication 18, dans lequel R₄ est CH₃.

20. Composé selon la revendication 19, dans lequel R₅ est CH₃.

21. Composé selon la revendication 20, dans lequel R₁ est H, et R₂ et R₃ sont chacun OH.

22. Procédé d'utilisation d'un composé de formule 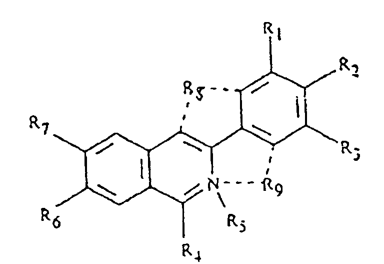 dans laquelle
R₁, R₂, R₃, R₆ et R₇ sont indépendamment H, OH ou un groupe alcoxy en C₁-C₈ ; R₂ et R₃ forment ensemble -OCH₂O- ; R₁ et R₂ forment ensemble -OCH₂O- ; ou R₆ et R₇ forment ensemble -OCH₂O- ;
R₄ est H ou un groupe alkyle en C₁-C₈,
R₅ est H, un groupe alkyle en C₁-C₈, ou est absent ; et
R₈ et R₉ sont indépendamment -CH=CH-, -(CH₂)₂-, ou
sont absents ;
à la condition que quand R₈ est -CH=CH- ou -(CH)₂-, R₉ soit absent et R₃ soit H ; et
quand R₉ est -CH=CH- ou -(CH₂)₂-, R₁ ou R₂ soit H, et R₅ et R₆ soient absents ;
ou un de ses sels acceptables d'un point de vue pharmaceutique ; pour préparer un médicament permettant d'inhiber la prolifération des cellules tumorales chez un mammifère présentant un cancer.

23. Composition pharmaceutique comprenant une quantité efficace d'un composé de formule 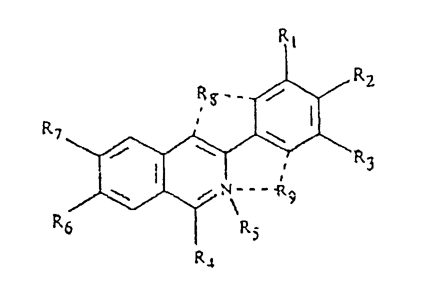 dans laquelle
R₁, R₂, R₆ et R₇ sont indépendamment H, OH ou un groupe alcoxy en C₁-C₈; R₃ est OH ou un groupe alcoxy en C₁-C₈; R₂ et R₃ forment ensemble -OCH₂O-; R₁ et R₂ forment ensemble -OCH₂O- ; ou R₆ et R₇ forment ensemble -OCH₂O- ;
R₄ est H ou un groupe alkyle en C₁-C₈,
R₅ est H, un groupe alkyle en C₁-C₈, ou est absent ; et
R₈ et R₉ sont indépendamment -CH=CH-, -(CH₂)₂-, ou sont absents ;
à la condition que quand R₈ est -CH=CH- ou -(CH)₂-, R₉ soit absent et R₃ soit H ; et
quand R₉ est -CH=CH- ou -(CH₂)₂-, R₁ ou R₂ soit H, et R₅ et R₈ soient absents ;
ou un de ses sels acceptables d'un point de vue pharmaceutique ; en combinaison avec un excipient acceptable d'un point de vue pharmaceutique.

24. Le composé acétosulfate de 8-méthyl-3,4,10,11-tétraméthoxydibenzo[a,g]quinolizinium ; acétosulfate de 8-méthyl-3,4-méthylènedioxy-10,11-diméthoxydibenzo[a,g]quinolizinium; acétosulfate de 5,6-dihydro-8-méthyl-3,4,10,11-tétraméthoxy-dibenzo[a,g]quinolizinium ; acétosulfate de 5,6-dihydro-8-méthyl-3,4-méthylènedioxy-10,11-diméthoxydibenzo[a,g]quinolizinium ; chlorure de 5,6-dihydro-3,4,10,11-tétraméthoxydibenzo[a,g]quinolizinium ; chlorure de 5,6-dihydro-3,4-méthylènedioxy-10,11-éthoxydibenzo[a,g]quino-lizinium ; chlorure de 3,4,10,11-tétraméthoxydibenzo[a,g]quinolizinium ; ou chlorure de 3,4-méthylènedioxy-10,11-diméthoxydibenzo[a,g]quinol-izinium ; ou l'un de ses sels acceptables d'un point de vue pharmaceutique.

25. Le composé 6,7-diméthoxy-1-méthyl-3-(3,4-méthylènedioxyphényl)isoquinoléine ; 6,7-dihydroxy-1-méthyl-3-(3,4-dihydroxyphényl)isoquinoléine ; méthosulfate de 6,7-diméthoxy-1,2-diméthyl-3-(3,4-méthylènedioxyphényl)isoquinoléinium ; ou méthosulfate de 6,7-dihydroxy-1,2-diméthyl-3-(3,4-dihydroxyphényl)isoquinoléinium; ou l'un de ses sels acceptables d'un point de vue pharmaceutique.

26. Composé de formule 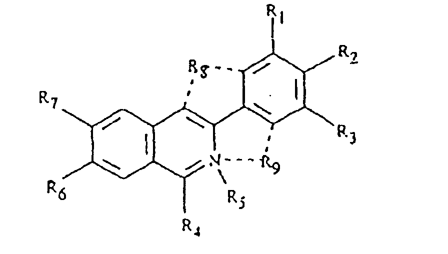 dans laquelle
R₁, R₂, R₆ et R₇ sont indépendamment H, OH ou un groupe alcoxy en C₁-C₈; R₁ et R₂ forment ensemble -OCH₂O- ; ou R₆ et R₇ forment ensemble -OCH₂O- ; et R₃ est H ;
R₄ est H ou un groupe alkyle en C₁-C₈;
R₅ est H, un groupe alkyle en C₁-C₈, ou est absent ;
R₈ est -(CH₂)₂- ; et R₉ est absent ; ou l'un de ses sels acceptables d'un point de vue pharmaceutique.

27. Composé de formule 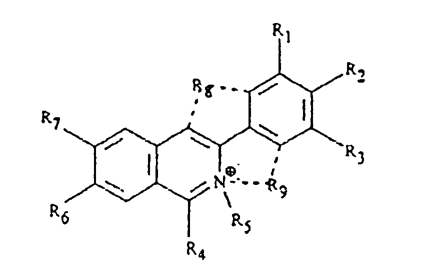 dans laquelle
R₁, R₂, R₃, R₆ et R₇ sont indépendamment H, OH ou un groupe alcoxy en C₁-C₈ ; R₂ et R₃ forment ensemble -OCH₂O- ; ou R₆ et R₇ forment ensemble -OCH₂O- ;
R₄ est H ou un groupe alkyle en C₁-C₈;
R₅ est absent ;
R₈ est absent ;
R₉ est -CH=CH- ;
à la condition que R₁ ou R₂ soit H ;
ou l'un de ses sels acceptables d'un point de vue pharmaceutique.

28. Composé de formule 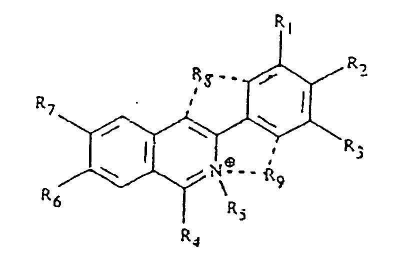 dans laquelle
R₁, R₂, R₃, R₆ et R₇ sont indépendamment H, OH ou un groupe alcoxy en C₁-C₈ ; R₂ et R₃ forment ensemble -OCH₂O- ; ou R₆ et R₇ forment ensemble -OCH₂O- ; ou
R₄ est H ou un groupe alkyle en C₁-C₈ ;
R₅ est absent ;
R₈ est absent ; et
R₉ est -(CH₂)₂- ;
à la condition que R₁ ou R₂ soit H ;
ou l'un de ses sels acceptables d'un point de vue pharmaceutique.

29. Composé de formule 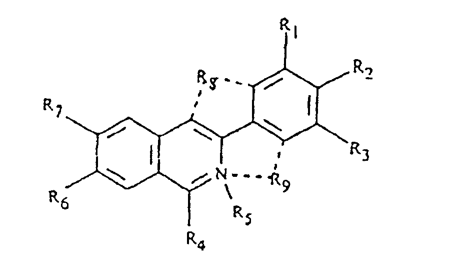 dans laquelle
R₃, R₆ et R₇ sont indépendamment H, OH ou un groupe alcoxy en C₁-C₈ ; ou R₆ et R₇ forment ensemble -OCH₂O- ;
R₁ et R₂ forment ensemble -OCH₂O- ;
R₄ est H ou un groupe alkyle en C₁-C₈;
R₅ est H, un groupe alkyle en C₁-C₈, ou est absent ; et
R₈ et R₉ sont chacun absents ; ou l'un de ses sels acceptables d'un point de vue pharmaceutique.

30. Composé de formule 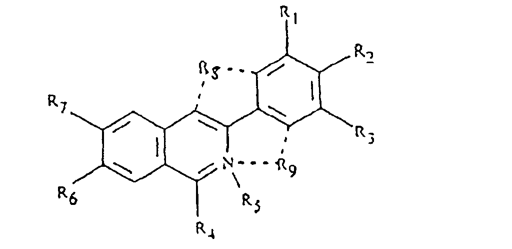 dans laquelle
R₁, R₆ et R₇ sont indépendamment H, OH ou un groupe alcoxy en C₁-C₈ ; ou R₆ et R₇ forment ensemble -OCH₂O- ;
R₂ et R₃ forment ensemble -OCH₂O- ;
R₄ est H ou un groupe alkyle en C₁-C₈;
R₅ est H, un groupe alkyle en C₁-C₈ ou est absent ; et
R₈ et R₉ sont chacun absents ;
ou l'un de ses sels acceptables d'un point de vue pharmaceutique.

31. Composé de formule 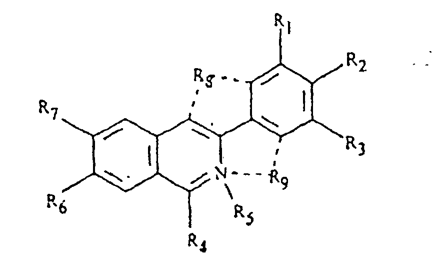 dans laquelle
R₁, R₂ et R₃ sont indépendamment H, OH ou un groupe alcoxy en C₁-C₈ ; R₂ et R₃ forment ensemble -OCH₂O- ; ou R₁ et R₂ forment ensemble -OCH₂O- ;
R₄ est H ou un groupe alkyle en C₁-C₈ ;
R₅ est H, un groupe alkyle en C₁-C₈, ou est absent ;
R₆ et R₇ forment ensemble -OCH₂O- ; et
R₈ et R₉ sont chacun absents ;
ou l'un de ses sels acceptables d'un point de vue pharmaceutique.
